# EUROPEAN PATENT APPLICATION

(11) **EP 2 336 340 A1**
(43) Date of publication of application: **22.06.2011**
(21) Application number: 09180145.6
(22) Date of filing: 21.12.2009
(51) Int. Cl.: C12P 5/00, C12P 5/02, C12N 9/88

(54) **Method for producing an alkene comprising the step of converting an alcohol by an enzymatic dehydration step**

(71) Applicant: Marliere, Philippe, 75020 Paris (FR)
(72) Inventor: Marliere, Philippe, 75020 Paris (FR)
(74) Representative: Heunemann, Dieter

(57) **Abstract**

Described is a method for producing an alkene comprising the step of converting an alcohol by an enzymatic dehydration step. The conversion preferably makes use of a prenyl isoflavonoid hydratase, in particular of a kievitone hydratase (EC 4.2.1.95) or of a phaseollidin hydratase (EC 4.2.1.97). Moreover, the present invention describes a recombinant organism which produces an alcohol and which expresses a hydratase enzyme which is capable of catalyzing the conversion of said alcohol into an alkene.

## Description

The present invention relates to a method for producing an alkene comprising the step of converting an alcohol by an enzymatic dehydration step. The conversion preferably makes use of a prenyl isoflavonoid hydratase, preferably of a kievitone hydratase (EC 4.2.1.95) or a phaseollidin hydratase (EC 4.2.1.97). Moreover, the present invention relates to a recombinant organism which produces an alcohol and which expresses a hydratase enzyme which is capable of catalyzing the conversion of said alcohol into an alkene.

A large number of chemical compounds are currently derived from petrochemicals. Alkenes (such as ethylene, propylene, the different butenes, or else the pentenes, for example) are used in the plastics industry, for example for producing polypropylene or polyethylene, and in other areas of the chemical industry and that of fuels. Ethylene, the simplest alkene, lies at the heart of industrial organic chemistry: it is the most widely produced organic compound in the world. It is used in particular to produce polyethylene, a major plastic. Ethylene can also be converted to many industrially useful products by reaction (e.g. by oxidation or halogenation). Propylene plays a similarly important role: its polymerization results in a plastic material, polypropylene. The technical properties of this product in terms of resistance, density, solidity, deformability, and transparency are unequalled. The worldwide market for polypropylene has grown continuously since its invention in 1954. Butylene exists in four forms, one of which, isobutylene, enters into the composition of methyl-tert-butyl-ether (MTBE), an anti-knock additive for automobile fuel. Isobutylene can also be used to produce isooctene, which in turn can be reduced to isooctane (2,2,4-trimethylpentane); the very high octane rating of isooctane makes it the best fuel for so-called "gasoline" engines. Amylene, hexene and heptene exist in many forms according to the position and configuration of the double bond. These products have real industrial applications but are less important than ethylene, propylene or butenes. All these alkenes are currently produced by catalytic cracking of petroleum products (or by a derivative of the Fischer-Tropsch process in the case of hexene, from coal or gas). Their production costs are therefore tightly linked to the price of oil. Moreover, catalytic cracking is sometimes associated with considerable technical difficulties which increase process complexity and production costs.

The production by a biological pathway of alkenes or other organic molecules that can be used as fuels or as precursors of synthetic resins is called for in the context of a sustainable industrial operation in harmony with geochemical cycles. The first generation of biofuels consisted in the fermentative production of ethanol, as fermentation and distillation processes already existed in the food processing industry. The production of second generation biofuels is in an exploratory phase, encompassing in particular the production of long chain alcohols (butanol and pentanol), terpenes, linear alkanes and fatty acids. Two recent reviews provide a general overview of research in this field: Ladygina et al. (Process Biochemistry 41 (2006),1001) and Wackett (Current Opinions in Chemical Biology 21 (2008), 187).

The production of ethylene by plants has long been known (Meigh et al. (Nature 186 (1960), 902)). According to the metabolic pathway elucidated, methionine is the precursor of ethylene (Adams and Yang (PNAS 76 (1979), 170)). Conversion of 2-oxoglutarate has also been described (Ladygina et al. (Process Biochemistry 41 (2006), 1001). Since a single ethylene molecule requires the previous production of a four- or five-carbon chain, the equipment and energy needs of all these pathways are unfavorable and do not bode well for their industrial application for alkene bioproduction.
Moreover, many microorganisms are capable of producing propylene, however, with an extremely low yield
The conversion of isovalerate to isobutylene by the yeast *Rhodotorula minuta* has been described (Fujii et al. (Appl. Environ. Microbiol. 54 (1988), 583)), but the efficiency of this reaction, less than 1 millionth per minute, or about 1 for 1000 per day, is far from permitting an industrial application. The reaction mechanism was elucidated by Fukuda et al. (BBRC 201 (1994), 516) and involves a cytochrome P450 enzyme which decarboxylates isovalerate by reduction of an oxoferryl group Fe^{V}=O. Large-scale biosynthesis of isobutylene by this pathway seems highly unfavorable, since it would require the synthesis and degradation of one molecule of leucine to form one molecule of isobutylene. Also, the enzyme catalyzing the reaction uses heme as cofactor, poorly lending itself to recombinant expression in bacteria and to improvement of enzyme parameters. For all these reasons, it appears very unlikely that this pathway can serve as a basis for industrial exploitation. Other microorganisms have been described as being marginally capable of naturally producing isobutylene from isovalerate; the yields obtained are even lower than those obtained with *Rhodotorula minuta* (Fukuda et al. (Agric. Biol. Chem. 48 (1984), 1679)).
Isoprene is produced at a significant level in higher plants, such as poplars. The production of isoprene in this context remains however low and the pathway which leads to isoprene production, which is based on the mevalonate-isopentenyl-pyrophosphate pathway, poorly complies with the demands for industrial scale production.

Thus, there is still a need for efficient and environmentally friendly methods of producing alkenes.

The present invention meets this demand by providing a method for producing an alkene comprising the step of converting an alcohol into an alkene by an enzymatic dehydration step.
The conversion preferably makes use of a hydratase enzyme, in particular of a prenyl isoflavonoid hydratase, preferably of a kievitone hydratase (EC 4.2.1.95) or a phaseollidin hydratase (EC 4.2.1.97).

The present invention teaches that it is possible to convert an alcohol enzymatically by the use of a hydratase into an alkene. The enzyme is preferably a prenyl isoflavonoid hydratase, more preferably a kievitone hydratase (EC 4.2.1.95) or a phaseollidin hydratase (EC 4.2.1.97).

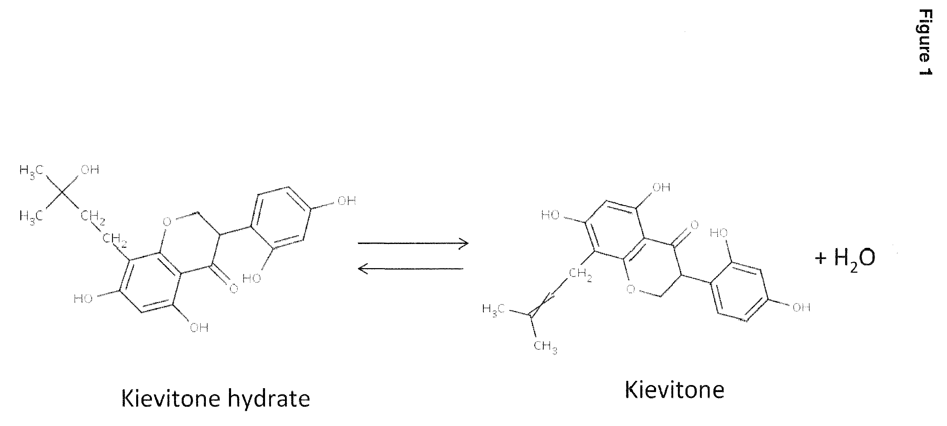

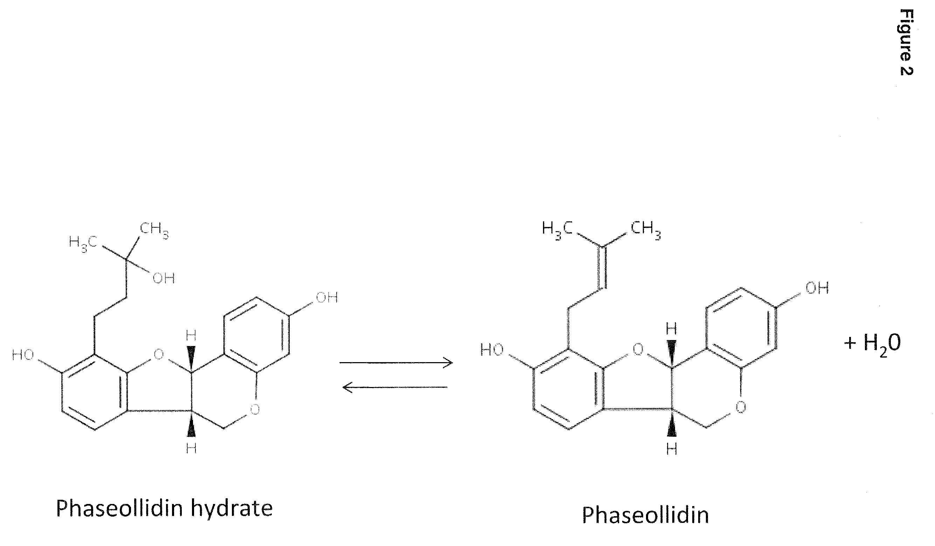

In particular, it has been found that an enzyme with the activity of a prenyl isoflavonoid hydratase, in particular a kievitone hydratase (EC 4.2.1.95) or a phaseollidin hydratase (EC 4.2.1.97), is not only capable of catalyzing the hydration of the corresponding prenyl isoflavonoid but is also capable of catalyzing the dehydration of alcohols and not only of longer alcohols but also of short chain alcohols. Thus, it was found that these enzymes can actually be used to convert alcohols into alkenes which allows the cost efficient and environmentally friendly production of alkenes in microorganisms or other organisms. Figure 1 shows the reaction catalyzed by kievitone hydratase and Figure 2 shows the reaction catalyzed by phaseollidin hydratase.

The alcohol to be used in the method according to the invention is a compound of formula (I):

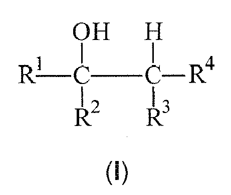

R¹, R², R³ and R⁴ are each independently selected from hydrogen or C₁₋₁₁ hydrocarbyl. More preferably, R¹ and R² are each C₁₋₁₁ hydrocarbyl, and R³ and R⁴ are each independently selected from hydrogen or C₁₋₁₁ hydrocarbyl.

Said hydrocarbyl is preferably selected from alkyl, alkenyl, alkynyl, cycloalkyl, cycloalkenyl, or aryl, more preferably selected from alkyl, alkenyl, alkynyl, or aryl, and even more preferably selected from alkyl, alkenyl, or aryl.

Said alkyl preferably comprises 1 to 11 carbon atoms, more preferably 1 to 8 carbon atoms, more preferably 1 to 6 carbon atoms, and even more preferably 1 to 4 (i.e. 1, 2, 3 or 4) carbon atoms.
Said alkenyl preferably comprises 2 to 11 carbon atoms, more preferably 2 to 8 carbon atoms, more preferably 2 to 6 carbon atoms, and even more preferably 2 to 4 (i.e. 2, 3 or 4) carbon atoms.
Said alkynyl preferably comprises 2 to 11 carbon atoms, more preferably 2 to 8 carbon atoms, more preferably 2 to 6 carbon atoms, and even more preferably 2 to 4 (i.e. 2, 3 or 4) carbon atoms.
Said cycloalkyl preferably comprises 3 to 11 carbon atoms, more preferably 3 to 8 carbon atoms, and even more preferably 3 to 6 (i.e. 3, 4, 5 or 6) carbon atoms.
Said aryl is preferably selected from phenyl or naphthyl, and is more preferably phenyl.

Accordingly, said C₁₋₁₁ hydrocarbyl is preferably selected from C₁₋₁₁ alkyl (in particular, C₁₋₆ alkyl or C₁₋₄ alkyl), C₂₋₁₁ alkenyl (in particular, C₂₋₆ alkenyl or C₂₋₄ alkenyl), C₂₋₁₁ alkynyl (in particular, C₂₋₆ alkynyl or C₂₋₄ alkynyl), C₃₋₁₁ cycloalkyl (in particular, C₃₋₆ cycloalkyl), C₃₋₁₁ cycloalkenyl (in particular, C₃₋₆ cycloalkenyl), phenyl or naphthyl. More preferably, said C₁₋₁₁ hydrocarbyl is selected from C₁₋₁₁ alkyl (in particular, C₁₋₆ alkyl or C₁₋₄ alkyl), C₂₋₁₁ alkenyl (in particular, C₂₋₆ alkenyl or C₂₋₄ alkenyl) or phenyl.

In a preferred embodiment, R¹, R², R³, and R⁴ are each independently selected from hydrogen, C₁₋₁₁ alkyl, C₂₋₁₁ alkenyl, C₂₋₁₁ alkynyl, C₃₋₁₁ cycloalkyl, C₃₋₁₁ cycloalkenyl, phenyl or naphthyl, and are more preferably each independently selected from hydrogen, C₁₋₆ alkyl, C₂₋₆ alkenyl or phenyl, and are even more preferably each independently selected from hydrogen or linear C₁₋₄ alkyl. In this embodiment, it is further preferred that R¹ and R² are different from hydrogen, i.e. that R¹ and R² each independently have the aforementioned meanings except for the option hydrogen.

Accordingly, in a particularly preferred embodiment, R¹ and R² are each independently selected from C₁₋₁₁ alkyl, C₂₋₁₁ alkenyl, C₂₋₁₁ alkynyl, C₃₋₁₁ cycloalkyl, C₃₋₁₁ cycloalkenyl, phenyl or naphthyl, and R³ and R⁴ are each independently selected from hydrogen, C₁₋₁₁ alkyl, C₂₋₁₁ alkenyl, C₂₋₁₁ alkynyl, C₃₋₁₁ cycloalkyl, C₃₋₁₁ cycloalkenyl, phenyl or naphthyl; more preferably, R¹ and R² are each independently selected from C₁₋₆ alkyl, C₂₋₆ alkenyl or phenyl, and R³ and R⁴ are each independently selected from hydrogen, C₁₋₆ alkyl, C₂₋₆ alkenyl or phenyl; even more preferably, R¹ and R² are each independently a linear C₁₋₄ alkyl, and R³ and R⁴ are each independently selected from hydrogen or linear C₁₋₄ alkyl.

In general, it is preferred that the compound of formula (I) comprises a total of 2 to 30 carbon atoms, more preferably 2 to 20 carbon atoms, more preferably 2 to 12 carbon atoms, more preferably 2 to 8 carbon atoms, more preferably 2 to 6 carbon atoms, even more preferably 2 to 5 carbon atoms, and most preferably 2, 3 or 4 carbon atoms.

Moreover, while primary, secondary and tertiary alcohols can generally be used in the method of the invention, it is preferred to use a tertiary alcohol. Accordingly, it is preferred that R¹ and R² comprised in the compound of formula (I) are each independently C₁₋₁₁ hydrocarbyl (or one of the preferred C₁₋₁₁ hydrocarbyl groups described and defined herein above).

It is particularly preferred that the alcohol (or, accordingly, the compound of formula (I)) is selected from: ethanol; propanol, including propan-1-ol (i.e. n-propanol) and propan-2-ol (i.e. isopropanol); butanol, including butan-1-ol (i.e. n-butanol), 2-methylpropan-1-ol (i.e. isobutanol), 2-methylpropan-2-ol (i.e. tert-butanol) and butan-2-ol (i.e. sec-butanol); or pentanol, including pentan-1-ol (i.e. n-pentanol or amyl alcohol), 3-methylbutan-1-ol (i.e. isopentanol or isoamyl alcohol), pentan-2-ol (i.e. sec-pentanol), pentan-3-ol, 2-methylbutan-1-ol, 3-methylbutan-2-ol (i.e. 1,2-dimethylpropanol) and 2-methylbutan-2-ol (i.e. 1,1-dimethylpropanol). Among the aforementioned butanols, 2-methylpropan-2-ol (i.e. tert-butanol) is most preferred; and among the aforementioned pentanols, 2-methylbutan-2-ol (i.e. 1,1-dimethylpropanol) is most preferred.

The following Table 1 gives an overview over some examples of alcohols to be employed in the method according to the invention and the resulting alkenes:

**Table 1**

| **Alcohol** | **Alkene** |
|---|---|
| ethanol | ethene |
| | (i.e. ethylene) |
| propan-1-ol | propene |
| (i.e. n-propanol) | (i.e. propylene) |
| propan-2-ol | propene |
| (i.e. isopropanol) | (i.e. propylene) |
| butan-1-ol | but-1-ene |
| (i.e. n-butanol) | (i.e. α-butylene) |
| 2-methylpropan-1-ol | 2-methylprop-1-ene |
| (i.e. isobutanol) | (i.e. isobutylene) |
| 2-methylpropan-2-ol | 2-methylprop-1-ene |
| (i.e. tert-butanol) | (i.e. isobutylene) |
| butan-2-ol | but-1-ene |
| (i.e. sec-butanol) | (i.e. α-butylene) |
| butan-2-ol | but-2-ene |
| (i.e. sec-butanol) | (i.e. β-butylene) |
| pentan-1-ol | pent-1-ene |
| (i.e. n-pentanol) | (i.e. amylene) |
| 3-methylbutan-1-ol | 3-methylbut-1-ene |
| (i.e. isopentanol) | |
| pentan-2-ol | pent-1-ene |
| (i.e. sec-pentanol) | (i.e. amylene) |
| pentan-2-ol | pent-2-ene |
| (i.e. sec-pentanol) | (i.e. β-amylene) |
| pentan-3-ol | pent-2-ene |
| | (i.e. β-amylene) |
| 2-methylbutan-l-ol | 2-methylbut-1-ene |
| 3-methylbutan-2-ol | 2-methylbut-2-ene |
| (i.e. 1,2-dimethylpropanol) | |
| 3-methylbutan-2-ol | 3-methylbut-1-ene |
| (i.e. 1,2-dimethylpropanol) | |
| 2-methylbutan-2-ol | 2-methylbut-1-ene |
| (i.e. 1,1-dimethylpropanol) | |
| 2-methylbutan-2-ol | 2-methylbut-2-ene |
| (i.e. 1,1-dimethylpropanol) | |
| 3-methylbut-3-en-1-ol | 2-methylbuta-1,3-diene |
| | (i.e. isoprene) |
| 2-methylbut-3-en-2-ol | 2-methylbuta-1,3-diene |
| | (i.e. isoprene) |
| 2-methylbut-3-en-2-ol | 3-methylbuta-1,2-diene |
| 2-methylbut-3-en-1-ol | 2-methylbuta-1,3-diene |
| | (i.e. isoprene) |
| 3-methylbut-3-en-2-ol | 2-methylbuta-1,3-diene |
| | (i.e. isoprene) |
| but-3-en-1-ol | buta-1,3-diene |
| but-3-en-2-ol | buta-1,3-diene |
| 1-phenylethanol | styrene |
| | (i.e. vinylbenzene) |
| 2-phenylethanol | styrene |
| | (i.e. vinylbenzene) |

In one particularly preferred embodiment the alcohol is ethanol and the produced alkene is ethene (i.e. ethylene). In another particularly preferred embodiment the alcohol is propan-2-ol (i.e. isopropanol) and the produced alkene is propene (i.e. propylene). Yet, in a further particularly preferred embodiment the alcohol is butanol and the produced alkene is butene (i.e. butylene). In this case butanol may be any of n-butanol, tert-butanol, sec-butanol or isobutanol. The produced alkene is either but-1-ene (i.e. α-butylene), 2-methylpropene (i.e. isobutylene), or both but-1-ene (i.e. α-butylene) and but-2-ene (i.e. β-butylene). In this context, n-butanol is most preferred for the production of but-1-ene (i.e. α-butylene) and tert-butanol is most preferred for the production of 2-methylpropene (i.e. isobutylene).

In a further particularly preferred embodiment the alcohol is any one of 3-methylbut-3-en-1-ol, 2-methylbut-3-en-2-ol, 2-methylbut-3-en-1-ol, 3-methylbut-3-en-2-ol, or mixtures thereof and the produced alkene is 2-methylbuta-1,3-diene (i.e. isoprene); in this embodiment, it is most preferred that the alcohol is 2-methylbut-3-en-2-ol and the produced alkene is 2-methylbuta-1,3-diene (i.e. isoprene). In another particularly preferred embodiment the alcohol is any one of but-3-en-1-ol, but-3-en-2-ol, or a mixture thereof and the produced alkene is buta-1,3-diene. In another particularly preferred embodiment the alcohol is any one of 1-phenylethanol, 2-phenylethanol, or a mixture thereof and the produced alkene is styrene (i.e. vinylbenzene). In another particularly preferred embodiment the alcohol is 2-methylbutan-2-ol and the produced alkene is 2-methylbut-2-ene.

It is to be understood that the alcohol to be used in the method according to the invention may also be a mixture of different compounds of formula (I).

With respect to the product of the method according to the invention, the term "alkene" refers to a compound comprising at least one carbon-to-carbon double bond and, in particular, refers to a compound of formula (II) 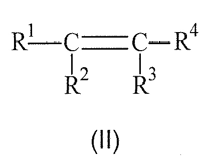 wherein R¹, R², R³, and R⁴ have the same meanings and preferred meanings as described and defined herein above for the compound of formula (I).

It is to be understood that all stereoisomers of the compound of formula (I) can be used in the method according to the invention, either in admixture or in pure or substantially pure form. Furthermore, the compound of formula (II) encompasses both cis-isomers and trans-isomers.

As used herein and unless defined otherwise, the term "hydrocarbyl" refers to a monovalent hydrocarbon group, i.e. a group consisting of carbon atoms and hydrogen atoms, which group is connected to the remainder of the compound of formula (I) via a carbon-to-carbon single bond and may be saturated or unsaturated, linear, branched or cyclic, aliphatic or aromatic. A "C₁₋₁₁ hydrocarbyl" denotes a hydrocarbyl having 1 to 11 carbon atoms.

As used herein and unless defined otherwise, the term "alkyl" refers to a monovalent saturated aliphatic (i.e. non-aromatic) acyclic hydrocarbon group which may be linear or branched and does not comprise any carbon-to-carbon double bond or any carbon-to-carbon triple bond.

As used herein and unless defined otherwise, the term "alkenyl" refers to a monovalent unsaturated aliphatic acyclic hydrocarbon group which may be linear or branched and comprises at least one carbon-to-carbon double bond while it does not comprise any carbon-to-carbon triple bond.

As used herein and unless defined otherwise, the term "alkynyl" refers to a monovalent unsaturated aliphatic acyclic hydrocarbon group which may be linear or branched and comprises at least one carbon-to-carbon triple bond and optionally one or more carbon-to-carbon double bonds.

As used herein and unless defined otherwise, the term "cycloalkyl" refers to a monovalent cyclic saturated aliphatic hydrocarbon group which does not comprise any carbon-to-carbon double bond or any carbon-to-carbon triple bond. Non-limiting examples of cycloalkyl groups are cyclopropyl, cyclobutyl, cyclopentyl or cyclohexyl.

As used herein and unless defined otherwise, the term "cycloalkenyl" refers to a monovalent cyclic unsaturated aliphatic hydrocarbon group which comprises at least one carbon-to-carbon double bond and does not comprise any carbon-to-carbon triple bond. Non-limiting examples of cycloalkyl groups are cyclopropenyl, cyclobutenyl, cyclopentenyl, cyclohexenyl or cyclohexadienyl.

As used herein and unless defined otherwise, the term "aryl" refers to a monovalent aromatic hydrocarbon group, including bridged ring and/or fused ring systems, containing at least one aromatic ring. "Aryl" may, for example, refer to phenyl, naphthyl or anthracenyl.

An enzyme with the activity of a hydratase is an enzyme which is capable of catalyzing the hydration-dehydration of C-O linkages.. In general, such an enzyme can also catalyze the reverse reaction, i.e. the dehydration. In the scope of dehydration such an enzyme catalyzes the elimination of oxygen and hydrogen from organic compounds in the form of water.
In general, in the context of the present invention any enzyme could be used which has hydratase activity. In a preferred embodiment the hydratase enzyme is a. prenyl isoflavonoid hydratase. A prenyl isoflavonoid hydratase is understood to be an enzyme which has the capacity to catalyse the addition of a water molecule to a prenyl isoflavonoid. The term "prenyl isoflavonoid" refers to a prenylated derivative of an isoflavonoid. Isoflavonoids are derivatives of isoflavone which belong to the class of the flavonoids. They are secondary plant metabolites which are, inter alia, involved in the pathogen defence of plants. Some examples of isoflavonoids are isoflavone, daidzein, genistein, prunetin, biochanin A, orobol, santal and pratensein. The prenylated isoflavonoid may in principle be any possible prenylated isoflavonoid. In a preferred embodiment the prenylated isoflavonoid is kievitone or phaseollidin.
Thus, in a particularly preferred embodiment the prenyl isoflavonoid hydratase is an enzyme which is capable of catalysing the addition of a water molecule to a prenyl isoflavonoid, preferably to kievitone or phaseollidin.
The prenyl isoflavonoid hydratase activity can be measured by incubating the corresponding enzyme with a prenyl isoflavonoid substrate under appropriate conditions and analysing the reaction products, e.g. by GC-MS analysis.
In a particularly preferred embodiment prenyl isoflavonoid hydratase is a kievitone hydratase (EC 4.2.1.95) or a phaseollidin hydratase (EC 4.2.1.97). In the context of the present invention the term "kievitone hydratase" or "an enzyme with the activity of a kievitone hydratase" or the term "phaseollidin hydratase" or "an enzyme with the activity of a phaseollidin hydratase" is understood to be an enzyme which is capable of catalyzing the hydration of a non-activated carbon-carbon bond. In particular, a kievitone hydratase (EC 4.2.1.95) is capable of converting kievitone into kievitone hydrate via hydration (see Figure 1). This enzymatic activity can be measured by incubating the enzyme with kievitone and analysing the reaction products, e.g. via thin layer or gas chromatography. It can in particular be assayed, e.g., by an assay as described in Li et al. (Mol. Plant-Microbe Interactions 3 (1995), 388-397). In brief, 5 ml of a suspension containing the enzyme are incubated for about 14 h with 50 µg kievitone with shaking at an appropriate temperature at which the enzyme shows activity (e.g., 22°C, 27 °C, 30°C or 37°C). The reaction is stopped and the products are analysed by thin layer chromatography. The occurrence of kievitone hydrate is indicative of kievitone hydratase activity. Alternatively, the activity can also be tested as described in Cleveland et al. (Physio!. Plant Pathol. 22 (1983), 129-142). In this assay 25 µg kievitone is mixed with the enzyme to be tested in 1 ml 50 mM potassium phosphate pH 6.0, and incubated for 2 to 6 h at an appropriate temperature (e.g., 22°C, 27°C, 30°C or 37°C) with shaking. Subsequently the products are characterized by thin layer chromatography.
A phaseollidin hydratase (EC 4.2.1.95) is capable of converting phaseollidin into phaseollidin hydrate via hydration (see Figure 2). This enzymatic activity can be measured by incubating the enzyme with phaseollidin and analysing the reaction products, e.g. via thin layer or gas chromatography. It can in particular be assayed, e.g., by an assay as described in Li et al. (Mol. Plant-Microbe Interactions 3 (1995), 388-397). In brief, 5 ml of a suspension containing the enzyme are incubated for about 14 h with 50 µg phaseollidin with shaking at an appropriate temperature at which the enzyme shows activity (e.g., 22°C, 27 °C, 30°C or 37°C). The reaction is stopped and the products are analysed by thin layer chromatography.
Preferably, in the context of the present application the term "kievitone hydratase" or "a protein/enzyme having the activity of a kievitone hydratase" refers to any enzyme which is classified in the EC number EC 4.2.1.95.
Preferably, in the context of the present application the term "phaseollidin hydratase" or "a protein/enzyme having the activity of a phaseollidin hydratase" refers to any enzyme which is classified in the EC number EC 4.2.1.97.
In principle any kievitone hydratase or phaseollidin hydratase enzyme can be used in the context of the present invention, in particular one from prokaryotic or eukaryotic organisms. Kievitone hydratase has been described from different organisms, e.g. from Fusarium solani (also referred to as Nectria haematococca; Li et al. (Mol. Plant-Microbe Interactions 3 (1995), 388-397); GeneBank accession number L39639). Sequences homologous to known kievitone hydratase sequences can be found in various organisms and are shown in SEQ ID NOs: 2 to 11.
These proteins constitute preferred embodiments of enzymes employed in the method according to the present invention.

In a preferred embodiment of the present invention the kievitone hydratase is an enzyme comprising an amino acid sequence selected from the group consisting of SEQ ID NOs: 1 to 11 or a sequence which is at least n % identical to any of SEQ ID NOs: 1 to 11 and having the activity of a kievitone hydratase with n being an integer between 10 and 100, preferably 10, 15, 20, 25, 30, 35, 40, 45, 50, 55, 60, 65, 70, 75, 80, 85, 90, 91, 92, 93, 94, 95, 96, 97, 98 or 99. The term "sequence identity" preferably means the same amino acid residues in the same N- to C-terminal direction.
Preferably, the degree of identity is determined by comparing the respective sequence with the amino acid sequence of any one of the above-mentioned SEQ ID NOs. When the sequences which are compared do not have the same length, the degree of identity preferably either refers to the percentage of amino acid residues in the shorter sequence which are identical to amino acid residues in the longer sequence or to the percentage of amino acid residues in the longer sequence which are identical to amino acid residues in the shorter sequence. The degree of sequence identity can be determined according to methods well known in the art using preferably suitable computer algorithms such as CLUSTAL.
When using the Clustal analysis method to determine whether a particular sequence is, for instance, 80% identical to a reference sequence default settings may be used or the settings are preferably as follows: Matrix: blosum 30; Open gap penalty: 10.0; Extend gap penalty: 0.05; Delay divergent: 40; Gap separation distance: 8 for comparisons of amino acid sequences. For nucleotide sequence comparisons, the Extend gap penalty is preferably set to 5.0.
Other algorithms which can be used for calculating sequence identity are those of Needleman and Wunsch or of Smith and Watermann. For sequence comparisons the program PileUp (Feng and Doolittle, J. Mol. Evolution 25 (1987), 351-360; Higgins et al., CABIOS 5 (1989), 151-153) or the programs Gap and Best Fit (Needleman and Wunsch, J. Mol. Biol. 48 (1970), 443-453; Smith and Waterman, Adv. Appl. Math. 2 (1981), 482-489) can be used, which are contained in the GCG software package (Genetics Computer Group, 575 Science Drive, Madison, Wisconsin, USA). Preferably, the settings which are used are the standard settings for sequence comparisons.
Preferably, the degree of identity is calculated over the complete length of the sequence.

The hydratase, preferably, kievitone hydratase or phaseollidin hydratase, employed in the process according to the invention can be a naturally occurring hydratase or it can be a hydratase which is derived from a naturally occurring hydratase, e.g. by the introduction of mutations or other alterations which, e.g., alter or improve the enzymatic activity, the stability, etc.
The term "hydratase" or "a protein/enzyme having the activity of a hydratase" in the context of the present application also covers enzymes which are derived from a hydratase, which are capable of dehydrating an alcohol as defined above into an alkene but which only have a low affinity to their natural substrate as a substrate or do no longer accept their natural substrate as a substrate. Such a modification of the preferred substrate of a hydratase allows to improve the conversion of the alcohol into the alkene and to reduce the production of unwanted by-product due to the action of the enzyme on its natural substrate.
The term "prenyl isoflavonoid hydratase" or "a protein/enzyme having the activity of a prenyl isoflavonoid hydratase" in the context of the present application also covers enzymes which are derived from a prenyl isoflavonoid hydratase, which are capable of dehydrating an alcohol as defined above into an alkene but which only have a low affinity to their natural prenyl isoflavonoid substrate as a substrate or do no longer accept their natural prenyl isoflavonoid substrate as a substrate. Such a modification of the preferred substrate of a prenyl isoflavonoid hydratase may allow to improve the conversion of the alcohol into the alkene and to reduce the production of unwanted by-product due to the action of the enzyme on its natural prenyl isoflavonoid substrate in case it is present.
Accordingly, the term "kievitone hydratase" or "a protein/enzyme having the activity of a kievitone hydratase" and the term "phaseollidin hydratase" or "a protein/enzyme having the activity of a phaseollidin hydratase" in the context of the present application also covers enzymes which are derived from a kievitone hydratase or a phaseollidin hydratase, which are capable of dehydrating an alcohol as defined above into an alkene but which only have a low affinity to their natural substrate kievitone or phaseollidin or do no longer accept their natural substrate kievitone or phaseollidin. Such a modification of the preferred substrate of a kievitone hydratase or phaseollidin hydratase allows to improve the conversion of the alcohol into the alkene and to reduce the production of unwanted by-product due to the action of the enzyme on its natural substrate kievitoneor phaseollidin.

Methods for modifying and/or improving the desired enzymatic activities of proteins are well-known to the person skilled in the art and include, e.g., random mutagenesis or site-directed mutagenesis and subsequent selection of enzymes having the desired properties or approaches of the so-called "directed evolution".
For example, for genetic engineering in prokaryotic cells, a nucleic acid molecule encoding the hydratase can be introduced into plasmids which permit mutagenesis or sequence modification by recombination of DNA sequences. Standard methods (see Sambrook and Russell (2001), Molecular Cloning: A Laboratory Manual, CSH Press, Cold Spring Harbor, NY, USA) allow base exchanges to be performed or natural or synthetic sequences to be added. DNA fragments can be connected to each other by applying adapters and linkers to the fragments. Moreover, engineering measures which provide suitable restriction sites or remove surplus DNA or restriction sites can be used. In those cases, in which insertions, deletions or substitutions are possible, *in vitro* mutagenesis, "primer repair", restriction or ligation can be used. In general, a sequence analysis, restriction analysis and other methods of biochemistry and molecular biology are carried out as analysis methods. The resulting hydratase variants are then tested for their enzymatic activity and in particular for their capacity to dehydrate alcohols and prefer them as a substrate rather than their natural substrate.
An assay for measuring the capacity of a hydratase to dehydrate an alcohol and to convert it into an alkene is describe in Examples, in particular Examples 2 to 8.

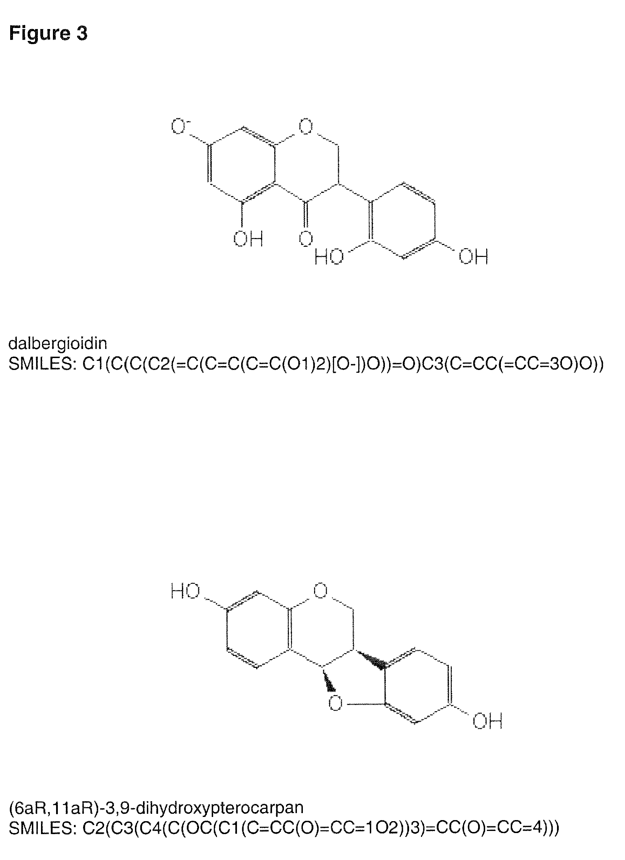

Methods for identifying variants with improved enzymatic properties as regards the production of alkenes may also be carried out in the presence of a "cofactor" which allows for a steric and/or electronic complementation in the catalytic site of the enzyme due to the fact that the alcohol used as a substrate may be shorter than the compound which functions as the natural substrate, kievitone or phaseollidin. The cofactor may depend on the natural substrate of the enzyme to be employed in the method according to the invention. In general, since in preferred embodiments of the invention short alcohols are employed for conversion into an alkene, the cofactor will generally be a cofactor corresponding to or mimicking the isoflavonoid portion of the prenyl isoflavonoid which naturally serves as the substrate for the hydratase employed. For example, in the case of kievitone hydratase, dalbergioidin or structural analogues thereof can be used as cofactors. In the case of phaseollidin hydratase, dihydroxypterocarpan or structural analogues thereof can be used as cofactors. The structural formulas of dalbergioidin and of dihydroxypterocarpan are shown in Figure 3.

The modified version of the hydratase accepting an alcohol as a substrate but having a low affinity to its natural substrate or no longer accepting its natural substrate, in particular kievitone or phaseollidin, may be derived from a naturally occurring hydratase, in particular kievitone or phaseollidin hydratase, or from an already modified, optimized or synthetically produced hydratase, in particular kievitone or phaseollidin hydratase.

The enzyme employed in the process according to the present invention can be a natural version of the protein or a synthetic protein as well as a protein which has been chemically synthesized or produced in a biological system or by recombinant processes. The enzyme may also be chemically modified, for example in order to improve its/their stability, resistance, e.g. to temperature, for facilitating its purification or its immobilization on a support. The enzyme may be used in isolated form, purified form, in immobilized form, as a crude or partially purified extract obtained from cells synthesizing the enzyme, as chemically synthesized enzyme, as recombinantly produced enzyme, in the form of microorganisms producing them etc.

The process according to the present invention may be carried out in vitro or in vivo. An in vitro reaction is understood to be a reaction in which no cells are employed, i.e. an acellular reaction.
For carrying out the process in vitro the substrates for the reaction and the enzyme are incubated under conditions (buffer, temperature, cofactors etc.) allowing the enzyme to be active and the enzymatic conversion to occur. The reaction is allowed to proceed for a time sufficient to produce alkene. The production of alkene can be detected by gas chromatography (GC) or GC/MS analysis.
The enzyme may be in any suitable form allowing the enzymatic reaction to take place. It may be purified or partially purified or in the form of crude cellular extracts or partially purified extracts. It is also possible that the enzyme is immobilized on a suitable carrier.
Since the alcohol used as a substrate may be shorter than the natural substrate used by the enzyme, e.g. kievitone or phaseollidin, it may be advantageous to add to the reaction mixture a "cofactor" which allows for a steric and/or electronic complementation in the catalytic site of the enzyme as mentioned above.

In general, if the alkene product is gaseous and scarcely soluble in water under the conditions of temperature at which the process is conducted, the equilibrium of the reaction catalyzed by the enzyme employed is shifted and the reaction goes to completion in the direction of the formation of the gaseous alkene, in particular if that gas is permanently removed from the reaction vessel.

In one particularly preferred embodiment, the hydratase enzyme used in the process according to the invention is a thermophilic hydratase, i.e. a hydratase which is capable of catalyzing the reaction at elevated temperatures. The term "elevated temperatures" means temperatures above 37°C. Such enzymes can either be isolated from corresponding thermophilic organisms, in particular bacteria or archaebacteria, or they can be obtained by mutagenizing available hydratase sequences and testing them for an increased enzymatic activity under increased temperature conditions. The advantage of using a hydratase which is functional at elevated temperatures is that the produced alkene can immediately go into the gaseous phase and can be constantly removed from the reaction thereby driving the reaction into the direction of dehydration. This advantage exists for all the produced alkenes which are in gaseous form at or below the temperature at which the reaction is carried out. Accordingly, in the method of the present invention the step of converting an alcohol by an enzymatic dehydration step into an alkene is preferably carried out at an elevated temperature (i.e. at a temperature above 37°C, including a temperature above 37°C and below 100°C, such as, e.g., at a temperature of 38°C, 40°C, 50°C, 70°C or 90°C) and the enzymatic dehydration step is catalyzed by a thermophilic hydratase as described herein above. The use of elevated temperatures also allows to produce longer alkenes in a manner that they directly degas from the reaction mixture, e.g. alkenes having 6 or 7 carbon atoms. Thus, in this preferred embodiment the alkene to be produced has preferably at least 4, even more preferably at least 5 even more preferably at least 6 and most preferably at least 7 carbon atoms. Moreover, it is preferred that the alkene does not have more than 12, 10 or 8 carbon atoms.

For carrying out the process in vivo use is made of a suitable organism/microorganism which is capable of expressing a hydratase enzyme as defined above. In principle, the substrate, i.e. the alcohol to be converted into the alkene, can be provided externally to the microorganism. However, in a preferred embodiment the organism/microorganism is also capable of producing the substrate, i.e. the alcohol to be converted.
Thus, in the case of this embodiment the method according to the invention is characterised in that the conversion of the alcohol into the alkene is realized in the presence of an organism/ microorganism capable of expressing a hydratase enzyme as defined above and preferably also capable of producing an alcohol which should be converted.
The term "which is capable of producing an alcohol" in the context of the present invention means that the organism/microorganism has the capacity to produce such an alcohol within the cell due to the presence of enzymes providing enzymatic activities allowing the production of such an alcohol from metabolic precursors. The organism/microorganism can be an organism/microorganism which naturally has the capacity to produce the corresponding alcohol or it can be an organism/microorganism which has been genetically modified so as to be capable of producing the corresponding alcohol.

For example, in one preferred embodiment of the present invention the alcohol to be converted into an alkene (in particular ethylene) is ethanol and the organism/microorganism employed in the method according to the invention is an organism/microorganism which is capable of producing ethanol. Examples for microorganism which naturally produce ethanol are yeast, in particular Saccharomyces species, Klyveromyces lactis and bacteria of the genus Zymomonas, most preferably Zymomonas mobilis. However, according to this embodiment any organism can be used which has the capacity to produce ethanol. In particular, it is also possible to use an organism/microorganism which has been genetically modified so as to confer to it the capacity to produce ethanol.

In another preferred embodiment of the present invention the alcohol to be converted into an alkene (in particular α-butylene) is n-butanol and the organism/microorganism employed in the method according to the invention is an organism/microorganism which is capable of producing n-butanol. Examples for microorganism which naturally produce n-butanol are bacteria of the genus Clostridium, preferably Clostridium acetobutylicum. However, it is also possible to use an organism/microorganism which has been genetically modified so as to confer to it the capacity to produce n-butanol.

In another preferred embodiment of the present invention the alcohol to be converted into an alkene (in particular isobutene) is isobutanol and the organism/microorganism employed in the method according to the invention is an organism/microorganism which is capable of producing isobutanol.

In another preferred embodiment of the present invention the alcohol to be converted into an alkene (in particular isobutene) is tert-butanol and the organism/microorganism employed in the method according to the invention is an organism/microorganism which is capable of producing tert-butanol.

In another preferred embodiment of the present invention the alcohol to be converted into an alkene (in particular isoprene) is 2-methyl-but-3-en-2-ol and the organism/microorganism employed in the method according to the invention is an organism/microorganism which is capable of producing 2-methyl-but-3-en-2-ol.

In another preferred embodiment of the present invention the alcohol to be converted into an alkene (in particular butadiene, such as e.g. buta-1,3-diene) is butenol (such as e.g. but-3-en-1-ol, but-3-en-2-ol, or a mixture thereof) and the organism/microorganism employed in the method according to the invention is an organism/microorganism which is capable of producing butenol.

In another preferred embodiment of the present invention the alcohol to be converted into an alkene (in particular styrene) is phenyl-ethanol (such as e.g. 1-phenylethanol, 2-phenylethanol, or a mixture thereof) and the organism/microorganism employed in the method according to the invention is an organism/microorganism which is capable of producing phenyl-ethanol.

In another preferred embodiment according to the present invention the alcohol to be converted into an alkene (in particular propylene) is n-propanol and the organism/microorganism employed in the method according to the invention is an organism/microorganism which is capable of producing n-propanol.

In yet a further preferred embodiment according to the present invention the alcohol to be converted into an alkene (in particular propylene) is isopropanol and the organism/microorganism employed in the method according to the invention is an organism/microorganism which is capable of producing isopropanol. Examples for microorganisms which are capable of producing isopropanol are acetone-producing bacteria which have been equipped with a secondary alcohol dehydrogenase which converts acetone into isopropanol. In particular, when a secondary alcohol dehydrogenase is produced in these bacteria the reaction (in the reverse orientation converts acetone into isopropanol that can then be further converted into isopropanol according to the method of the present invention. Corresponding E. coli cells are described in Jojima et al. (Appl. Microbiol. Biotechnol. 77 (2008), 1219-1224) and in Hanai et al. (Appl. Environ. Microbiol. 73 (2007), 7814-7818). A source for the secondary alcohol dehydrogenase is, e.g. Clostridium beijerinckii.

Acetone is produced by certain microorganisms, such as Clostridium acetobutylicum, Clostridium beijerinckii, Clostridium cellulolyticum, Bacillus polymyxa and Pseudomonas putida. The synthesis of acetone is best characterized in Clostridium acetobutylicum. It starts out with a reaction (reaction step 1) in which two molecules of acetyl-CoA are condensed into acetoacetyl-CoA. This reaction is catalyzed by acetyl-CoA acetyltransferase (EC 2.3.1.9). Acetoacetyl-CoA is then converted into acetoacetate by a reaction with acetic acid or butyric acid resulting also in the production of acetyl-CoA or butyryl-CoA (reaction step 2). This reaction is catalyzed e.g. by acetoacetylCoA transferase (EC 2.8.3.8). AcetoacetylCoA transferase is known from various organisms, e.g. from E. coli in which it is encoded by the atoAD gene or from Clostridium acetobutylicum in which it is encoded by the ctfAB gene. However, also other enzymes can catalyze this reaction, e.g. 3-oxoacid CoA transferase (EC 2.8.3.5) or succinate CoA ligase (EC 6.2.1.5).
Finally, acetoacetate is converted into acetone by a decarboxylation step (reaction step 3) catalyzed by acetoacetate decarboxylase (EC 4.1.1.4).
The above described reaction steps 1 and 2 and the enzymes catalyzing them are not characteristic for the acetone synthesis and can be found in various organism. In contrast, reaction step 3 which is catalyzed by acetoacetate decarboxylase (EC 4.1.1.4) is only found in those organisms which are capable of producing acetone. The prior art already describes microorganisms which have been genetically modified so as to be able to produce acetone. In particular genes from, e.g., Clostridium acetobutylicum have been introduced into E. coli thereby allowing the synthesis of acetone in E. coli, a bacterium which naturally does not produce acetone (Bermejo et al., Appl. Environ. Microbiol. 64 (1998); 1079-1085; Hanai et al., Appl. Environ. Microbiol. 73 (2007), 7814-7818). In particular Hanai et al. (loc. cit.) shows that it is sufficient to introduce a nucleic acid sequence encoding an acetoacetate decarboxylase (such as that from Clostridium acetobutylicum) in order to achieve acetone production in E. coli indicating that the endogenous enzymes in E. coli catalyzing the above-mentioned reaction steps 1 and 2 (i.e. the expression products of the E. coli atoB and atoAD genes) are sufficient to provide substrate for the acetone production.

In one preferred embodiment the organism employed in the method according to the invention is an organism, preferably a microorganism, which naturally has the capacity to produce acetone and furthermore expresses a secondary alcohol dehydrogenase so as to convert acetone into isopropanol which can then be further converted into propylene. Thus, preferably the microorganism belongs to the genus Clostridium, Bacillus or Pseudomonas, more preferably to the species Clostridium acetobutylicum, Clostridium beijerinckii, Clostridium cellulolyticum, Bacillus polymyxa or Pseudomonas putida. However, it is, of course, also possible to use an organism/microorganism which has been genetically modified so as to confer to it the capacity to produce acetone. The genes responsible for acetone production have been cloned and also methods for transferring these genes into other organisms, such as E. coli, to confer to them the capacity to produce acetone have already been described.

In a preferred embodiment, the organism employed in the method according to the invention is an organism, preferably a microorganism, which has the capacity to produce the respective alcohol to be converted into the corresponding alkene and which is recombinant in the sense that it has further been genetically modified so as to express a hydratase enzyme as defined above. The term "recombinant" in one embodiment means that the organism is genetically modified so as to contain a foreign nucleic acid molecule encoding a hydratase enzyme as defined above. In a preferred embodiment the organism has been genetically modified so as to contain a foreign nucleic acid molecule encoding a hydratase enzyme as defined above. The term "foreign" in this context means that the nucleic acid molecule does not naturally occur in said organism/microorganism. This means that it does not occur in the same structure or at the same location in the organism/microorganism. In one preferred embodiment, the foreign nucleic acid molecule is a recombinant molecule comprising a promoter and a coding sequence encoding the hydratase enzyme in which the promoter driving expression of the coding sequence is heterologous with respect to the coding sequence. Heterologous in this context means that the promoter is not the promoter naturally driving the expression of said coding sequence but is a promoter naturally driving expression of a different coding sequence, i.e., it is derived from another gene, or is a synthetic promoter or a chimeric promoter. Preferably, the promoter is a promoter heterologous to the organism/microorganism, i.e. a promoter which does not naturally occur in the respective organism/microorganism. Even more preferably, the promoter is an inducible promoter. Promoters for driving expression in different types of organisms, in particular in microorganisms, are well known to the person skilled in the art.
In another preferred embodiment the nucleic acid molecule is foreign to the organism/microorganism in that the encoded hydratase enzyme is not endogenous to the organism/microorganism, i.e. is naturally not expressed by the organism/microorganism when it is not genetically modified. In other words, the encoded hydratase enzyme is heterologous with respect to the organism/microorganism.
The term "recombinant" in another embodiment means that the organism is genetically modified in the regulatory region controlling the expression of a hydratase enzyme as defined above which naturally occurs in the organism so as to lead to an increase in expression of the respective enzyme in comparison to a corresponding non-genetically modified organism. The meaning of the term high "higher expression" is described further below.
Such a modification of a regulatory region can be achieved by methods known to the person skilled in the art. One example is to exchange the naturally occurring promoter by a promoter which allows for a higher expression or to modify the naturally occurring promoter so as to show a higher expression. Thus, in this embodiment the organism contains in the regulatory region of the gene encoding an enzyme as defined above a foreign nucleic acid molecule which naturally does not occur in the organism and which leads to a higher expression of the enzyme in comparison to a corresponding non-genetically modified organism.
The foreign nucleic acid molecule may be present in the organism/microorganism in extrachromosomal form, e.g. as plasmid, or stably integrated in the chromosome. A stable integration is preferred.
In another preferred embodiment the organism/microorganism is characterized in that the expression/activity of a hydratase enzyme as defined above is higher in the organism/microorganism genetically modified with the foreign nucleic acid molecule in comparison to the corresponding non-genetically modified organism/microorganism. A "higher" expression/activity means that the expression/activity of the hydratase enzyme in the genetically modified microorganism is at least 10%, preferably at least 20%, more preferably at least 30% or 50%, even more preferably at least 70% or 80% and particularly preferred at least 90% or 100% higher than in the corresponding non-genetically modified organism/microorganism. In even more preferred embodiments the increase in expression/activity may be at least 150%, at least 200% or at least 500%. In particularly preferred embodiments the expression is at least 10-fold, more preferably at least 100-fold and even more preferred at least 1000-fold higher than in the corresponding non-genetically modified organism/microorganism.
The term "higher" expression/activity also covers the situation in which the corresponding non-genetically modified organism/microorganism does not express a corresponding hydratase enzyme so that the corresponding expression/activity in the non-genetically modified organism/microorganism is zero.
Methods for measuring the level of expression of a given protein in a cell are well known to the person skilled in the art. In one embodiment, the measurement of the level of expression is done by measuring the amount of the corresponding protein. Corresponding methods are well known to the person skilled in the art and include Western Blot, ELISA etc. In another embodiment the measurement of the level of expression is done by measuring the amount of the corresponding RNA. Corresponding methods are well known to the person skilled in the art and include, e.g., Northern Blot.
Methods for measuring the enzymatic activity of the hydratase enzyme are known in the art and have already been described above.

Methods for preparing an organism which is genetically modified so as to produce a hydratase as described above, preferably a microorganism, are well known in the art. Thus, generally, the organism/microorganism is transformed with a DNA construct allowing expression of the respective enzyme in the microorganism. Such a construct normally comprises the coding sequence in question linked to regulatory sequences allowing transcription and translation in the respective host cell, e.g. a promoter and/or enhancer and/or transcription terminator and/or ribosome binding sites etc.

The term "organism" as used in the context of the present invention refers in general to any possible type of organism, in particular eukaryotic organisms, prokaryotic organisms and archaebacteria. The term includes animal, plants, fungi, bacteria and archaebacteria. The term also includes isolated cells or cell aggregates of such organisms, like tissue or calli.
In one preferred embodiment, the organism is a microorganism. The term "microorganism" in the context of the present invention refers to prokaryotic cells, in particular bacteria, as well as to fungi, such as yeasts, and also to algae and archaebacteria. In one preferred embodiment, the microorganism is a bacterium. In principle any bacterium can be used. Preferred bacteria to be employed in the process according to the invention are bacteria of the genus Bacillus, Clostridium, Pseudomonas, Zymomonas or Escherichia. In a particularly preferred embodiment the bacterium belongs to the genus Escherichia and even more preferred to the species Escherichia coli.
In another preferred embodiment the microorganism is a fungus, more preferably a fungus of the genus Saccharomyces, Schizosaccharomyces, Aspergillus or Trichoderma and even more preferably of the species Saccharomyces cerevisiae, Schizosaccharomyces pombe, Aspergillus niger or of the species Trichoderma reesei.
In still another preferred embodiment the microorganism is a photosynthetically active microorganism such as bacteria which are capable of carrying out photosynthesis or micro-algae.
In a particularly preferred embodiment the microorganism is an algae, more preferably an algae belonging to the diatomeae.

If microorganisms are used in the context of the method of the present invention, it is also conceivable to carry out the method according to the invention in a manner in which two types of microorganisms are employed, i.e. one type which produces the alcohol which should be converted into an alkene and one type which uses the alcohol produced by the first type of microorganisms to convert it with the help of a hydratase enzyme as defined herein above into the respective alkene.

When the process according to the invention is carried out in vivo by using microorganisms providing the respective enzyme activity, the microorganisms are cultivated under suitable culture conditions allowing the occurrence of the enzymatic reaction. The specific culture conditions depend on the specific microorganism employed but are well known to the person skilled in the art. The culture conditions are generally chosen in such a manner that they allow the expression of the genes encoding the enzymes for the respective reactions. Various methods are known to the person skilled in the art in order to improve and fine-tune the expression of certain genes at certain stages of the culture such as induction of gene expression by chemical inducers or by a temperature shift.

In another preferred embodiment the organism employed in the method according to the invention is an organism which is capable of photosynthesis, such as a plant or microalgae. In principle any possible plant can be used, i.e. a monocotyledonous plant or a dicotyledonous plant. It is preferable to use a plant which can be cultivated on an agriculturally meaningful scale and which allows to produce large amounts of biomass. Examples are grasses like Lolium, cereals like rye, barley, oat, millet, maize, other starch storing plants like potato or sugar storing plants like sugar cane or sugar beet. Conceivable is also the use of tobacco or of vegetable plants such as tomato, pepper, cucumber, egg plant etc. Another possibility is the use of oil storing plants such as rape seed, olives etc. Also conceivable is the use of trees, in particular fast growing trees such as eucalyptus, poplar or rubber tree (Hevea brasiliensis).

In a particularly preferred embodiment the organism/microorganism employed in the method according to the invention is an organism/microorganism which is thermophilic in the sense that it can survive and catalyze the dehydration of the alcohol in question at elevated temperatures. The term "elevated" temperature means a temperature over 37°C. Examples for such organism/microorganism are bacteria of the genus Thermus, e.g. Thermus thermophilus or Thermus aquaticus, or bacteria of the genus Clostridium, such as Clostridium thermocellum. Other examples are microorganisms which are extremely heat-tolerant, e.g. microorganisms of the genus Thermotoga, such as Thermotoga maritime, or microorganisms of the genus Aquifex, such as Aquifex aeolicus.

The present invention also relates to an organism, preferably a microorganism, which is characterized by the following features:
(a) it is capable of producing an alcohol; and
(b) it expresses a hydratase enzyme which is capable of catalyzing the conversion of the alcohol into an alkene.

As regards the source, nature, properties, sequence etc. of the hydratase enzyme expressed in the organism according to the invention, the same applies as has been set forth above in connection with the method according to the invention.

In one preferred embodiment, the organism according to the invention is an organism, preferably a microorganism, which naturally has the capacity to produce the alcohol, i.e., feature (a) mentioned above is a feature which the organism, preferably microorganism, shows naturally. In a particularly preferred embodiment the alcohol is ethanol, n-propanol, isopropanol, n-butanol, isobutanol, tert-butanol, 2-methylbut-3-en-2-ol, but-3-en-1-ol, but-3-en-2-ol, 1-phenylethanol or 2-phenylethanol.
As regards organisms/microorganisms producing such alcohols, the same as set forth above in connection with the method according to the invention applies.

In another preferred embodiment, the organism, preferably microorganism, according to the invention is a genetically modified organism/microorganism derived from an organism/microorganism which naturally does not produce the respective alcohol but which has been genetically modified so as to produce said alcohol, i.e. by introducing the gene(s) necessary for allowing the production of the alcohol in the organism/microorganism. In principle any organism/microorganism can be genetically modified in this way. The enzymes responsible for the synthesis of the respective alcohols are generally known. Genes encoding corresponding enzymes are known in the art and can be used to genetically modify a given organism, preferably microorganism so as to produce the alcohol.

In a further preferred embodiment the organism, preferably a microorganism, according to the invention is genetically modified so as to express a hydratase enzyme which is capable of catalyzing the conversion of an alcohol into an alkene. In this context, the term "recombinant" means in a first aspect that the organism contains a foreign nucleic acid molecule encoding a corresponding hydratase enzyme. The term "foreign" in this context means that the nucleic acid molecule does not naturally occur in said organism/microorganism. This means that it does not occur in the same structure or at the same location in the organism/microorganism. In one preferred embodiment, the foreign nucleic acid molecule is a recombinant molecule comprising a promoter and a coding sequence encoding said hydratase enzyme in which the promoter driving expression of the coding sequence is heterologous with respect to the coding sequence. Heterologous in this context means that the promoter is not the promoter naturally driving the expression of said coding sequence but is a promoter naturally driving expression of a different coding sequence, i.e., it is derived from another gene, or is a synthetic promoter or a chimeric promoter. Preferably, the promoter is a promoter heterologous to the organism/microorganism, i.e. a promoter which does naturally not occur in the respective organism/microorganism. Even more preferably, the promoter is an inducible promoter. Promoters for driving expression in different types of organisms, in particular microorganisms, are well known to the person skilled in the art.
In another preferred embodiment the nucleic acid molecule is foreign to the organism/microorganism in that the encoded hydratase enzyme is not endogenous to the organism/microorganism, i.e. is naturally not expressed by the organism/microorganism when it is not genetically modified. In other words, the encoded hydratase enzyme is heterologous with respect to the organism/microorganism.
The term "recombinant" in another aspect means that the organism is genetically modified in the regulatory region controlling the expression of an enzyme as defined above which naturally occurs in the organism so as to lead to an increase in expression of the respective enzyme in comparison to a corresponding non-genetically modified organism. The meaning of the term high "higher expression" is described further below.
Such a modification of a regulatory region can be achieved by methods known to the person skilled in the art. One example is to exchange the naturally occurring promoter by a promoter which allows for a higher expression or to modify the naturally occurring promoter so as to show a higher expression. Thus, in this embodiment the organism contains in the regulatory region of the gene encoding an enzyme as defined above a foreign nucleic acid molecule which naturally does not occur in the organism and which leads to a higher expression of the enzyme in comparison to a corresponding non-genetically modified organism.

In a further preferred embodiment the organism/microorganism is characterized in that the expression/activity of the hydratase enzyme is higher in the organism/microorganism genetically modified with the foreign nucleic acid molecule in comparison to the corresponding non-genetically modified organism/microorganism. A "higher" expression/activity means that the expression/activity of the hydratase enzyme in the genetically modified organism/microorganism is at least 10%, preferably at least 20%, more preferably at least 30% or 50%, even more preferably at least 70% or 80% and particularly preferred at least 90% or 100% higher than in the corresponding non-genetically modified organism/microorganism. In even more preferred embodiments the increase in expression/activity may be at least 150%, at least 200% or at least 500%. In particularly preferred embodiments the expression is at least 10-fold, more preferably at least 100-fold and even more preferred at least 1000-fold higher than in the corresponding non-genetically modified organism/microorganism.
The term "higher" expression/activity also covers the situation in which the corresponding non-genetically modified organism/microorganism does not express a corresponding hydratase enzyme so that the corresponding expression/activity in the non-genetically modified organism/microorganism is zero.
Methods for measuring the level of expression of a given protein in a cell are well known to the person skilled in the art. In one embodiment, the measurement of the level of expression is done by measuring the amount of the corresponding protein. Corresponding methods are well known to the person skilled in the art and include Western Blot, ELISA etc. In another embodiment the measurement of the level of expression is done by measuring the amount of the corresponding RNA. Corresponding methods are well known to the person skilled in the art and include, e.g., Northern Blot.
Methods for measuring the enzymatic activity of the hydratase enzyme are known in the art and have already been described above.
The term "organism" as used in the context of the present invention refers in general to any possible type of organism, in particular eukaryotic organisms, prokaryotic organisms and archaebacteria. The term includes animal, plants, fungi, bacteria and archaebacteria. The term also includes isolated cells or cell aggregates of such organisms, like tissue or calli.
In one preferred embodiment, the organism is a microorganism. The term "microorganism" in the context of the present invention refers to prokaryotic cells, in particular bacteria, as well as to fungi, such as yeasts, and also to algae and archaebacteria. In one preferred embodiment, the microorganism is a bacterium. In principle any bacterium can be used. Preferred bacteria to be employed in the process according to the invention are bacteria of the genus Bacillus, Clostridium, Pseudomonas, Zymomonas or Escherichia. In a particularly preferred embodiment the bacterium belongs to the genus Escherichia and even more preferred to the species Escherichia coli.
In another preferred embodiment the microorganism is a fungus, more preferably a fungus of the genus Saccharomyces, Schizosaccharomyces, Aspergillus or Trichoderma and even more preferably of the species Saccharomyces cerevisiae, Schizosaccharomyces pombe, Aspergillus niger or of the species Trichoderma reesei.
In still another preferred embodiment the microorganism is a photosynthetically active microorganism such as bacteria which are capable of carrying out photosynthesis or micro-algae.
In a particularly preferred embodiment the microorganism is an algae, more preferably an algae from the genus belonging to the diatomeae.

In another preferred embodiment the organism according to the invention is an organism which is capable of photosynthesis, such as a plant or micro-algae. In principle, it can be any possible plant, i.e. a monocotyledonous plant or a dicotyledonous plant. It is preferably a plant which can be cultivated on an agriculturally meaningful scale and which allows to produce large amounts of biomass. Examples are grasses like Lolium, cereals like rye, barley, oat, millet, maize, other starch storing plants like potato or sugar storing plants like sugar cane or sugar beet. Conceivable is also the use of tobacco or of vegetable plants such as tomato, pepper, cucumber, egg plant etc. In another preferred embodiment the plant is an oil storing plants such as rape seed, olives etc. Also conceivable is the use of trees, in particular fast growing trees such as eucalyptus, poplar or rubber tree (Hevea brasiliensis).

In a particularly preferred embodiment the organism/microorganism employed in the method according to the invention is an organism/microorganism which is thermophilic in the sense that it can survive and catalyze the dehydration of the alcohol in question at elevated temperatures. The term "elevated" temperature means a temperature over 37°C. Examples for such organism/microorganism are bacteria of the genus Thermus, e.g. Thermus thermophilus or Thermus aquaticus, or bacteria of the genus Clostridium, such as Clostridium thermocellum. Other examples are microorganisms which are extremely heat-tolerant, e.g. microorganisms of the genus Thermotoga, such as Thermotoga maritime, or microorganisms of the genus Aquifex, such as Aquifex aeolicus.

The present invention also relates to the use of an organism, preferably a microorganism, which is characterized by the following features:
(a) it is capable of producing an alcohol; and
(b) it expresses a hydratase enzyme which is capable of catalyzing the conversion of said alcohol into an alkene
   for the production of an alkene.
I.e., the present invention also relates to the use of an organism/microorganism according to the invention for the production of an alkene from the respective alcohol.

The present invention also relates to a composition comprising an organism according to the present invention.

Moreover, the present invention also relates to a composition comprising (i) an alcohol; and (ii) a hydratase enzyme which is capable of catalyzing the conversion of said alcohol into an alkene.
For the preferred embodiments of the enzyme the same applies as has already been set forth above in connection with the method and the organism according to the invention.

Moreover, the present invention also relates to the use of a hydratase enzyme which is capable of catalyzing the conversion of an alcohol into an alkene for the production of an alkene as defined herein-above from said alcohol.
For the preferred embodiments of the enzyme the same applies as has already been set forth above in connection with the method and the organism according to the invention.

Finally, the present invention also relates to the use of an alcohol for the production of an alkene, comprising the enzymatic conversion of the alcohol by a hydratase enzyme which is capable of catalyzing the conversion of the alcohol into an alkene. In a preferred embodiment the enzymatic conversion is achieved by an enzyme as described above in connection with the method according to the invention, more preferably with a prenyl isoflavonoid hydratase, even more preferably a kievitone hydratase or a phaseollidin hydratase and most preferably the conversion is achieved by the use of an organism according to the invention.
- **Figure 1**:: Figure 1 shows the reaction catalyzed by kievitone hydratase.
- **Figure 2**:: Figure 2 shows the reaction catalyzed by phaseollidin hydratase.
- **Figure 3**:: Figure 3 shows the structure of possible cofactors for kievitone hydratase, dalbergioidin (SMILES: C1(C(C(C2(=C(C=C(C=C(O1)2)[O- ])O))=O)C3(C=CC(=CC=3O)O))), and for phaseollidin hydratase, dihydroxypterocarpan (SMILES: C2(C3(C4(C(OC(C1(C=CC(O)=CC=102))3)=CC(O)=CC=4)))

The following Examples serve to illustrate the invention.

### Example 1: Cloning, expression and purification of a collection of kievitone hydratases and or phaseollidin hydratases

A library of genes encoding enzymes homologous to the kievitone hydratase (EC 4.2.1.95) of Fusarium solani (SEQ ID NO:1) is cloned, expressed and purified.

### Cloning, bacterial culture and expression of proteins

Amino-acid sequences corresponding to proteins homologous to the kievitone hydratase (EC 4.2.1.95) of Fusarium solani (SEQ ID NO:1) are obtained from sequence database using the BLAST algorithm. A set of 20 sequences are chosen for maximizing genetic distance. In order to obtain a high level of expression in *E. coli*, the DNA sequences inferred from these protein sequences are generated taking in consideration the Codon Adaptation Index, as defined by Sharp and Li (Nucl. Acids Res. 15 (1987), 1281-1295).
The optimized sequences are synthesized by oligonucleotide concatenation (Geneart) and cloned in a pET25 expression vector, with the addition of a methionine initiation codon and a 6-histidine tag added at the N-terminal end immediately preceding the first methionine of the original protein sequence. Competent *E. coli* BL21(DE3) cells (Novagen) are transformed with these vectors by heat shock. The cells are grown with shaking (160 rpm) at 30°C in TB medium containing 0.5 M sorbitol, 5 mM betain, 100 µg/ml ampicillin until reaching an OD at 600 nm comprised between 0.8 and 1. Isopropyl-B-D-thiogalactopyranoside (IPTG) is then added to a final concentration of 1 mM and protein expression is continued at 20°C overnight (approximately 16 h). The cells are collected by centrifugation at 4°C, 10.000 rpm for 20 min and the pellets are frozen at -80°C.

### Cell lysis

1.6 g of cells are thawed on ice and resuspended in 5 ml of 50 mM Na₂HPO₄ pH 8 containing 300 mM NaCl, 5 mM MgCl₂, 1 mM DTT. Twenty microliters of lysonase (Novagen) are added. Cells are incubated for 10 min at room temperature and then returned to ice for 20 min. Cell lysis is completed by sonication for 3x 5 min in an ultrasound water bath at 0°C; samples are homogenized between pulses. The bacterial extracts are then clarified by centrifugation at 4°C, 10,000 rpm for 20 min.

### Protein purification and concentration (PROTINO kit)

The clarified bacterial lysates are loaded on a PROTINO-1000 Ni-IDA column (Macherey-Nagel) allowing adsorption of 6-His-tagged proteins. Columns are washed and the enzymes of interest are eluted with 4 ml of 50 mM Na₂HPO₄ pH 8 containing 300 mM NaCl, 5 mM MgCl₂, 1 mM DTT, 250 mM imidazole. Eluates are then concentrated in Amicon Ultra-4 10 kDa cells (Millipore) to a final volume of 250 µl. Proteins are quantified by the Bradford method. SDS-PAGE analyses are conducted in parallel: a main band is clearly visible around 65kD, and the purity of the protein is estimated to over 80%.

### Example 2: Measuring the activity of purified kievitone hydratases or phaseollidin hydratases towards ethanol

### Enzymatic reactions

Reactions are run in 1 ml of buffer containing 20mM Tris (pH 8) and 50 mM NaCl, and various concentration of ethanol, ranging from 100mM to 5M. 1 mg of enzyme is added to the sample. The enzyme-free control reactions are carried out in parallel. The 1 ml reactions are placed in 2 ml vials (Interchim) and sealed with a teflon/silica/teflon septum (Interchim). Reactions are incubated with shaking at 27°C for 72 h.

### Analysis of reactions

The gas present in the headspace is collected using a gas syringe equipped with an anti-backup mechanism. The gas sample is analyzed by gas chromatography (GC) coupled with a flame ionization detector.
Column: BPX5 (SGE)
GC: Hewlett Packard 5890 series II
For each chromatogram, the presence of a peak is analyzed precisely, and compared to negative control chromatograms and chromatograms obtained when injecting pure ethylene (Sigma). A significant production of a gas corresponding to the elution time of ethylene is observed in the case of several enzymatic preparations.

The identity of the gas if further confirmed using GC/MS analysis, giving the precise physical signature of ethylene.
Column: BPX5 (SGE)
GC/MS: MSD 5973 (HP)

### Example 3: Measuring the activity of purified kievitone hydratases or phaseollidin hydratases towards propanol isomers

The same protocol as in Example 2 is followed to assay the activity of the collection of 20 enzymes towards propan-1-ol and propan-2-ol (isopropanol) instead of ethanol. A signal indicating the significant production of a gas having the same elution time than propylene is identified in the case of several enzymes.
The identity of propylene is further confirmed in GC/MS analyses.

### Example 4: Measuring the activity of purified kievitone hydratases or phaseollidire hydratases towards linear butanol isomers

The same protocol as in Example 2 is followed to assay the activity of the collection of 20 enzymes towards butan-1-ol (n-butanol), butan-2-ol (sec-butanol) instead of ethanol. A signal indicating the significant production of a gas having the same elution time than linear butene (but-1-ene or the cis or trans isomers of but-2-ene) is identified in the case of several enzymes.
The identity of the gas is further confirmed in GC/MS analyses. In the case of several enzymes, only one of the three linear butenes is obtained. In some other cases, a mix of two or the three compounds is obtained.

### Example 5: Measuring the activity of purified kievitone hydratases or phaseollidin hydratases towards branched butanol isomers

The same protocol as in Example 2 is followed to assay the activity of the collection of 20 enzymes towards 2-methylpropan-1-ol (isobutanol) or 2-methylpropan-2-ol (*tert-*butanol) instead of ethanol. A signal indicating the significant production of a gas having the same elution time than 2-methylprop-1-ene (isobutene) is identified in the case of several enzymes.
The identity of the gases is further confirmed in GC/MS analyses.

### Example 6: Measuring the activity of purified "kievitone hydratases or phaseollidin hydratases towards isopentenol

The same protocol as in Example 2 is followed to assay the activity of the collection of 20 enzymes towards isopentenol isomers (3-methylbut-3-en-l-ol, 3-methylbut-3-en-2-ol, 2-methylbut-3-en-1-ol, 2-methylbut-3-en-2-ol) instead of ethanol. A signal indicating the significant production of a gas having the same elution time than 2-methylbuta-1,3-diene (isoprene) is identified in the case of several enzymes.
The identity of isoprene is further confirmed in GC/MS analyses.

### Example 7: Measuring the activity of purified kievitone hydratases or phaseollidin hydratases towards branched butenol isomers

The same protocol as in Example 2 is followed to assay the activity of the collection of 20 enzymes towards but-3-en-l-ol or but-3-en-2-ol instead of ethanol. A signal indicating the significant production of a gas having the same elution time than buta-1,3-diene is identified in the case of several enzymes.
The identity of buta-1,3-diene is further confirmed in GC/MS analyses.

### Example 8: Measuring the activity of purified kievitone hydratases or phaseollidin hydratases towards phenyl-ethanol isomers

The same protocol as in Example 2 is followed to assay the activity of the collection of 20 enzymes towards 1-phenylethanol or 2-phenylethanol instead of ethanol. As the expected product, vinylbenzene (styrene), is liquid at ambient temperature, what is harvested here is not gas from the headspace, but liquid from the reaction mix. Two microliter of such reaction mix are injected in the GC apparatus, and the signal indicating the significant production of a gas having the same elution time than styrene is identified in the case of several enzymes.
The identity of the gas is further confirmed in GC/MS analyses.

### Example 9: Optimization of reaction conditions by using a cofactor

The same assays as in Examples 2 to 8 are carried out, but a cofactor such as dalbergioidin or dihydroxypterocarpan is added to the reaction mix.
Vials containing the buffer, an enzyme preparation, an alcohol and 1 mg/mL of a cofactor (dalbergioidin or dihydroxypterocarpan) are prepared and tested following the same protocol as described in Example 2.
An increase in the rate of conversion of the alcohol into the corresponding alkene is observed in several cases.

### Example 10: Bacteria synthesizing ethylene from glucose

Ethanol producing E.coli bacteria are transformed using a plasmid encoding an enzyme having shown activity in converting ethanol into ethylene, as described in example 2. The transformed bacteria are then grown in mineral medium containing 20g/L glucose under aeration at 30°C during 24 hours. The production of ethylene in the headspace is measured by taking a sample using a gas syringe, and using a GC apparatus as described in Example 2.

### Example 11: Bacteria synthesizing propylene from glucose

*E. coli* bacteria engineered to produce propan-2-ol as described in Jojima et al. (2008, Appl. Microbial. Biotechnol. 77:1219-1224) are transformed by a plasmid encoding an enzyme of the prenyl isoflavonoid family, e.g. a kievitone hydratase or a phaseollidin hydratase, showing activity in converting propan-2-ol into propylene, as described in Example 3. The transformed bacteria are then grown in mineral medium containing 20g/L glucose under aeration at 30°C during 24 hours. The production of propylene in the headspace is measured by taking a sample using a gas syringe, and using a GC apparatus as previously described.

### Example 12: Bacteria synthesizing isobutene from glucose

*E. coli* bacteria engineered to produce 2-methylpropan-1-ol (isobutanol), as described in Connor and Liao, Curr. Opin. Biotechnol. 20, 2009:307, are transformed using a plasmid encoding an enzyme having shown activity in converting branched butanol isomers into isobutene, as described in Example 5. The transformed bacteria are then grown in mineral medium containing 20g/L glucose under aeration at 30°C during 24 hours. The production of isobutene in the headspace is measured by taking a sample using a gas syringe, and using a GC apparatus as previously described.

### Example 13: Bacteria synthesizing isoprene from glucose

*E. coli* bacteria first engineered to produce 3-methylbut-3-en-1-ol are further transformed using a plasmid encoding an enzyme having shown activity in converting branched methyl-butenol isomers into isoprene, as described in Example 6. The transformed bacteria are then grown in mineral medium containing 20g/L glucose under aeration at 30°C during 24 hours. The production of isoprene in the headspace is measured by taking a sample using a gas syringe, and using a GC apparatus as previously described.

### Example 14: Bacteria synthesizing butadiene from glucose

*E. coli* bacteria first engineered to produce but-3-en-1-ol are transformed using a plasmid encoding an enzyme having shown activity in converting butenol isomers into butadiene, as described in Example 7. The transformed bacteria are then grown in mineral medium containing 20g/L glucose under aeration at 30°C during 24 hours.
The production of butadiene in the headspace is measured by taking a sample using a gas syringe, and using a GC apparatus as previously described.

### Example 15: Bacteria synthesizing styrene from glucose

*E. coli* bacteria first engineered to produce 1-phenylethanol are transformed using a plasmid encoding an enzyme having shown activity in converting branched butenol isomers into vinylbenzene (styrene), as described in Example 8. The transformed bacteria are then grown in mineral medium containing 20g/L glucose under aeration at 30°C during 24 hours. Two microliters of the liquid fraction of the reaction mix is harvested using a syringe, and injected in a GC apparatus as previously described to identify the presence of styrene.

## Claims

1. A method for producing an alkene, the method comprising a step of converting an alcohol by an enzymatic dehydration step into an alkene, wherein:
the alcohol is a compound of formula (I) 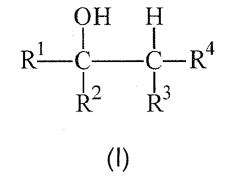 wherein R¹, R², R³ and R⁴ are each independently selected from hydrogen or C₁₋₁₁ hydrocarbyl; and
the alkene is a compound of formula (II) 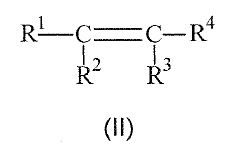 wherein R¹, R², R³, and R⁴ have the same meanings as defined for the compound of formula (I).

2. The method of claim 1 wherein the enzymatic dehydration step is catalyzed by a hydratase.

3. The method of claim 1 or 2 wherein the enzymatic dehydration step is catalyzed by a prenyl isoflavonoid hydratase.

4. The method of claim 3 wherein the prenyl isoflavonoid hydratase is a kievitone hydratase (EC 4.2.1.95).

5. The method of claim 3 wherein the prenyl isoflavonoid hydratase is a phaseollidin hydratase (EC 4.2.1.97).

6. The method of claim 4 or 5 wherein the prenyl isoflavonoid hydratase has an amino acid sequence as set forth in any one of SEQ ID NOs: 1 to 11 or a sequence having at least 15% sequence identity to any one of SEQ ID NO: 1 to 12 preferably 30%, 40%, 50%, 60%, 80% or 90%.

7. The method of any one of claims 1 to 6 wherein:
(i) the alcohol is ethanol and the alkene is ethene; or
(ii) the alcohol is propan-1-ol and the alkene is propene; or
(iii) the alcohol is propan-2-ol and the alkene is propene; or
(iv) the alcohol is butan-1-ol and the alkene is but-1-ene; or
(v) the alcohol is 2-methylpropan-1-ol and the alkene is 2-methylprop-1-ene; or
(vi) the alcohol is 2-methylpropan-2-ol and the alkene is 2-methylprop-1-ene; or
(vii) the alcohol is butan-2-ol and the alkene is but-1-ene; or
(viii) the alcohol is butan-2-ol and the alkene is but-2-ene; or
(ix) the alcohol is 3-methylbut-3-en-1-ol and the alkene is 2-methylbuta-1,3-diene; or
(x) the alcohol is 2-methylbut-3-en-2-ol and the alkene is 2-methylbuta-1,3-diene; or
(xi) the alcohol is 2-methylbut-3-en-1-ol and the alkene is 2-methylbuta-1,3-diene; or
(xii) the alcohol is 3-methylbut-3-en-2-ol and the alkene is 2-methylbuta-1,3-diene; or
(xiii) the alcohol is but-3-en-1-ol and the alkene is buta-1,3-diene; or
(xiv) the alcohol is but-3-en-2-ol and the alkene is buta-1,3-diene; or
(xv) the alcohol is 1-phenylethanol and the alkene is styrene; or
(xvi) the alcohol is 2-phenylethanol and the alkene is styrene.

8. The method of any one of claims 1 to 7 wherein the step of converting an alcohol by an enzymatic dehydration step into an alkene is carried out at a temperature above 37°C and the enzymatic dehydration step is catalyzed by a thermophilic hydratase.

9. The method of claim 8 wherein the alkene comprises 6 or 7 carbon atoms.

10. The method of any one of claims 1 to 9 wherein the step of converting an alcohol by an enzymatic dehydration step into an alkene is realized in the presence of an organism capable of expressing an enzyme as defined in any one of claims 2 to 6.

11. The method of claim 10 wherein the microorganism is furthermore capable of producing the alcohol to be converted.

12. A recombinant organism which is **characterized by** the following features:
(a) it is capable of producing an alcohol as defined in claim 1; and
(b) it expresses a hydratase enzyme which is capable of catalyzing the conversion of said alcohol into an alkene.

13. Use of an organism as defined in claim 12 for the production of an alkene.

14. A composition comprising an organism of claim 12.

15. A composition comprising
(i) an alcohol as defined in claim 1; and
(ii) a hydratase enzyme which is capable of catalyzing the conversion of said alcohol into an alkene

16. Use of an enzyme as defined in any one of claims 2 to 6 for the production of an alkene.


SEQUENCE LISTING

<110>  Marliere , Philippe

<120>  Method for producing an alkene comprising the step of converting
       an alcohol by an enzymatic dehydration step

<130>  R3188 EP S3

<160>  11

<170>  PatentIn version 3.5

<210>  1
<211>  350
<212>  PRT
<213>  Nectria haematococca

<400>  1

Met Met Ile Ser Ser Val Leu Val Ala Gly Val Val Ala Val Ser Ala
1               5                   10                  15

Ala Leu Ala Ser Lys His Pro Lys Gln Tyr Ser Phe Lys Pro Glu Asp
            20                  25                  30

Ala Glu Thr Ile Trp Asn Gly Asp Ile Pro Val Leu Tyr Asp Phe Gly
        35                  40                  45

Asp Ser Gln Ser Ala Ser Tyr Ser Gly Ser Trp Trp Thr Ser Ser Tyr
    50                  55                  60

Ile Thr Gly Thr Asn Gly Glu Gln Tyr Leu Val Ile Ser His Tyr Leu
65                  70                  75                  80

Asp Thr Pro Val Phe Thr Tyr Phe Arg Ala Ser Thr Leu Asn Leu Glu
                85                  90                  95

Thr Leu Asp Tyr Asn Gln Phe Ile Thr Leu Gly Asn Asn Thr Ala Asn
            100                 105                 110

Ser Thr Thr Leu Asp Val Lys Val Gly Asp Asn Gly Ile Gln Ser Leu
        115                 120                 125

Thr Ala Asp Asn Ile Ser Gln Gln Arg Ala Tyr Ala Asn Asp Glu Asn
    130                 135                 140

Val Thr Phe Asp Ile Thr Phe Asp Ala Thr Ser Arg Val Ile Ser Asn
145                 150                 155                 160

Ala Gly Ala Gly Val Phe Gln Phe Gly Pro Ser Ile Thr Tyr Glu Trp
                165                 170                 175

Gly Leu Pro Asn Cys Arg Thr Gln Gly Ser Val Thr Asp Thr Gly Gly
            180                 185                 190

Lys Asn Ile Thr Val Asp Pro Ala Lys Ser Phe Thr Trp Tyr Asp Arg
        195                 200                 205

Gln Trp Gly Thr Ala Ala Val Thr Ser Gly Asn Trp Thr Trp Phe Gln
    210                 215                 220

Met His Ile Pro Glu Thr Ser Tyr Lys Leu Ser Val Trp Ile Ile Asp
225                 230                 235                 240

Asn Asp Val Thr Asn Gln Phe Ser Arg Phe Ala Thr Ile Arg Gly Asp
                245                 250                 255

Asn Asp Glu Phe Gln Val Leu Pro Leu Glu Trp Lys Pro Ile Tyr Asp
            260                 265                 270

Arg Thr Tyr Gln Ser Thr Ala Ala Asp Ile Leu Tyr Pro Leu Asp Trp
        275                 280                 285

Glu Leu Asp Ile Ser Gly Phe Gly Val Phe Gln Leu Ser Ser Ile Leu
    290                 295                 300

Asp Asp Gln Glu Ile Val Gly Thr Thr Ala Ile Gln Thr Ala Tyr Glu
305                 310                 315                 320

Gly Phe Val Thr Phe Asn Gly Thr Val His Asn Lys Lys Val Gln Gly
                325                 330                 335

Tyr Gly Leu Val Glu Val Val Tyr Ser Asn Trp Glu Ser Leu
            340                 345                 350

<210>  2
<211>  347
<212>  PRT
<213>  Emericella nidulans

<400>  2

Met Arg Pro Ala Ile Leu Ala Ala Phe Ser Thr Leu Pro Ala Ala Ala
1               5                   10                  15

Lys Ala Thr Tyr Pro Phe Ala Pro Glu Thr Phe Asp Gly Ser Tyr Lys
            20                  25                  30

Asp Ile Gly Leu Pro Thr Ile Tyr Asp Leu Ser Ala Thr Gln Ser Thr
        35                  40                  45

Asn Tyr Asn Gly Ser Trp Ala Thr Gly Ser Trp Ile Thr Ser Val Ser
    50                  55                  60

Gly Gly Gln Tyr Phe Val Val Ser His Tyr Val Asn Asp Gly Ile His
65                  70                  75                  80

Asp Val Tyr Arg Ser Ser Ile Leu Asp Leu Ser Ser Leu Lys Tyr Arg
                85                  90                  95

Tyr Phe Phe Gln Ala Gly Asn Gly Ser Tyr Thr Ala Ser Pro Pro Ser
            100                 105                 110

His Leu Lys Ala Gly Val Gly Lys Gly Asn Gly Phe Glu Gly Ile Ser
        115                 120                 125

Asn Asp Asn Tyr Thr Thr Met Arg Val Gln Ser Ser His Pro Asn Val
    130                 135                 140

Thr Phe Asp Leu Thr Tyr His Ala Thr Thr Lys Pro Leu Ile Asn Gly
145                 150                 155                 160

Gly Ala Gly Val Val Met Leu Gly Ala Ser Glu Ser Lys Gln Trp Ser
                165                 170                 175

Leu Pro Ala Cys Trp Thr Asn Gly Phe Leu Ile Val Gly Asp Glu Gln
            180                 185                 190

Ile Pro Ile Asp Pro Lys Arg Ser Leu Thr Trp Tyr Asp Arg Gln Trp
        195                 200                 205

Gly Thr Gly Gly Leu Thr Asn Trp Thr Trp Tyr Gly Leu His Ile Pro
    210                 215                 220

Lys Thr Gly His Val Leu Ser Ile Trp Thr Gly Asp Thr Asp Ala Asp
225                 230                 235                 240

Arg Ala Ala Pro Ile Thr Pro Val Arg Phe Ala Thr Val Arg Asn Ala
                245                 250                 255

Tyr Gly Ala Gln Thr Val Cys Asn Ile Thr Trp Ile Pro Asp Leu Ser
            260                 265                 270

His Ile Phe His Ser Asp Ser Thr Asn Lys Ser Tyr Pro Leu Ala Trp
        275                 280                 285

Thr Val Glu Ile Pro Ser Tyr Asp Ala Ile Ile Lys Val Lys Ser Arg
    290                 295                 300

Thr Glu Asn Gln Leu Asn Thr Gly Ser His Gly Ser Glu Pro Glu Ala
305                 310                 315                 320

Tyr Asn Gly Phe Val Thr Phe Ala Gly Gln Phe Gln Gly Thr Glu Thr
                325                 330                 335

Glu Gly Phe Gly Ile Val Glu Ile Val Tyr Leu
            340                 345

<210>  3
<211>  370
<212>  PRT
<213>  Aspergillus oryzae

<400>  3

Met Ala Ile Leu Phe Lys Leu Ser Leu Pro Ile Trp Ala Leu Ala Val
1               5                   10                  15

Val Gln Ala Thr Ser Ser His Cys Ser His Lys Gln Ser Ser His His
            20                  25                  30

Thr Ala Ala Tyr Asp Phe His Pro Ser Gln Ala Gly Ile Gln Thr Ala
        35                  40                  45

Gln Leu Ile Asp Pro Leu Pro Leu Pro Tyr Asp Phe Asn Thr Ser Asn
    50                  55                  60

Ser Glu Met Ala Asp Ser Ser Asn Ser Trp Trp Met Ser Ser Tyr Leu
65                  70                  75                  80

Thr Gly Ser Asp Asn His Gln Tyr Met Val Leu Ser His Val Leu Ala
                85                  90                  95

Thr Asp Thr Phe Gly Leu Tyr Arg Gly Gly Ile Tyr Asp Ile Thr Glu
            100                 105                 110

Pro Ala Tyr Ser Leu Phe Ser Glu Met Thr Thr Gln Asn Leu Thr Ala
        115                 120                 125

His Ser Gln Asp Gly Lys Phe Asn Ile Arg Thr Asp Asp Phe Phe Phe
    130                 135                 140

Gly Ser Ala Leu Ala Asn Asn Ser Ile Thr Lys Leu Arg Thr Val Ser
145                 150                 155                 160

Asn Arg Thr Asn Leu Gln Phe Asp Leu Thr Tyr Glu Leu Thr Ala Pro
                165                 170                 175

Val Leu Phe Asn Ala Gly Leu Gly Gly Leu Phe Gln Phe Gly Pro Asp
        180                 185                 190

Gln Thr Gly Glu Trp Ser Met Pro Ala Gly Lys Thr Ser Gly Ser Leu
        195                 200                 205

Val Phe Asn Gly Lys Lys Ile Thr Val Asp Thr Lys Arg Ser Gln Thr
    210                 215                 220

Trp Tyr Asp Arg Gln Trp Asn Val Gly Pro Ala Pro Thr Ser His Thr
225                 230                 235                 240

Trp Thr Trp Phe Gln Leu His Ile Asn Asn Gly Glu Ser Asp Glu Lys
                245                 250                 255

Glu Leu Val Ser Leu Trp Met Tyr Asp Ser Asp Val Lys Gly His Arg
            260                 265                 270

Gln Trp Ala Thr Thr Gln Ser Lys Ala Gly Ile Asn Ile Val Gln Ser
        275                 280                 285

Val Pro Thr Val Glu Pro Phe Gly Asn Ser Trp Thr Ser Pro His Thr
    290                 295                 300

Asn Ala Thr Tyr His Gln Ala Trp Lys Ile Val Leu Gln Asp Ser Thr
305                 310                 315                 320

Thr Leu Thr Val Lys Thr Thr Tyr Glu Asp Gln Glu Leu Trp Ala Ala
                325                 330                 335

Arg Gly Phe Ala Thr Tyr Glu Gly Phe Val Thr Val Asn Gly Thr Asp
            340                 345                 350

Ala Asp Gly Lys Cys Val Thr Gly Phe Gly Leu Val Glu Ile Gln Gly
        355                 360                 365

His Phe
    370

<210>  4
<211>  366
<212>  PRT
<213>  Aspergillus terreus (strain NIH 2624 / FGSC A1156)

<400>  4

Met Asp Ile Ala Arg Gly Val Leu Leu Trp Ala Leu Thr Gln Val Leu
1               5                   10                  15

Tyr Glu Ile Pro Glu Gln Val Ser Met Gln Phe Gly Ser Arg Val Ile
            20                  25                  30

Arg Pro Arg Pro Arg Ile Pro Pro Cys Thr His Ile Pro Leu Pro Ser
        35                  40                  45

Arg Gly Arg Arg Asn Ile Leu Ala Gly Lys Ser Thr Thr Pro Ala Leu
    50                  55                  60

Asn Pro Ser Thr Pro Leu Leu Phe Asn Phe Ser His Ser Lys Leu Leu
65                  70                  75                  80

Asn Thr Thr Ser Ser Thr Asn Ser Tyr Trp Thr Ser Ser Tyr Ile Thr
                85                  90                  95

Asn Thr Thr Asn Glu Gln Tyr Leu Leu Ile Ser His Leu Leu Val Gly
            100                 105                 110

Pro Ser Asp Arg Ser Trp Tyr Arg Ala Ser Ala Leu Asn Leu Ala Asn
        115                 120                 125

Pro Ser Asn Tyr Thr Thr Thr Val Thr Ser Ser Thr Ala Pro Val Phe
    130                 135                 140

Gln Pro Gly Pro Gln Leu Arg Ala Thr Met Arg Thr Phe Ser Ala Asp
145                 150                 155                 160

Ser Pro Val Leu Thr Phe Asp Leu Thr Tyr Ala Ala Thr Ser Thr Ala
                165                 170                 175

Leu Val Asn Gly Gly Met Gly Leu Phe Ala Phe Gly Ser Gly Arg Thr
            180                 185                 190

Tyr Glu Trp Gly Leu Pro Asn Cys Val Thr Thr Gly Ser Ala Arg Ile
        195                 200                 205

Asn Gly Ala Pro Leu Arg Val Asp Pro Ala Arg Ser Phe Thr Trp Phe
    210                 215                 220

Asp Arg Gln Trp Ile Glu Ala Ala Ala Ala Ala Ser Trp Thr Arg Phe
225                 230                 235                 240

Glu Leu His Val Ala Gly Ser Arg Asp Arg Leu Ser Val Trp Ala Ile
                245                 250                 255

Asp Ser Gly Glu Gly Gly Gly Pro Gly Val Arg Phe Ala Thr Ile Arg

260                 265                 270

Arg Gly Asp Gly Ala Gln Leu Val Val Pro Val Gly Phe Arg Ala Asp
        275                 280                 285

Tyr Thr Arg Gln Trp Tyr Ser Gln Ala Ser Gly Lys Leu Tyr Pro Leu
    290                 295                 300

Asp Trp Val Val Thr Val Gly Glu Val Gly Val Phe Arg Ile Ala Ser
305                 310                 315                 320

Ile Val Gly Asp Gln Glu Ile Ala Gly Gly Ser Ala Phe Pro Thr Ala
                325                 330                 335

Tyr Glu Gly Phe Val Thr Phe Asp Gly Arg Phe Glu Gly Gln Glu Val
            340                 345                 350

Gly Gly Phe Gly Val Val Glu Ile Ile Phe Ser Gly Glu Ser
        355                 360                 365

<210>  5
<211>  412
<212>  PRT
<213>  Sclerotinia sclerotiorum (strain ATCC 18683 / 1980 / Ss-1) GN=SS1G_11539
PE=4 SV=1

<400>  5

Met Ser Gly Ile Leu Glu Cys Pro Ile Asp Phe Ala Thr Ser Val Tyr
1               5                   10                  15

Arg Asn Gly Cys Thr Val Val Ile Cys Asn Leu Lys Asp Arg Pro Asp
            20                  25                  30

Tyr Leu Leu Cys Arg Val Val Asn Glu Ser Ser Gly Leu Thr Ser Asp
        35                  40                  45

Ala Asp His Val Thr Tyr Val Ala Arg Asn Leu Asn Asn Leu Ser Gln
    50                  55                  60

Tyr Ser Asn Gly Lys Leu Glu Gln Asn Leu Leu His Val Tyr His Val
65                  70                  75                  80

Val Leu Ala Ala Tyr Ala Pro Cys Gly Lys Gln Cys Ser Ala Glu Leu
                85                  90                  95

Pro Asn Gln Tyr Ser Leu Pro Glu Ser Gln Leu Val Thr Pro Pro Val
            100                 105                 110

Asn Ser Phe Trp Ser Thr His Phe Leu Thr Ser Ala Asn Ser Thr Glu
        115                 120                 125

Tyr Val Leu Leu Ser His Ala Phe Val Leu Pro Ser Gly Thr Val Thr
    130                 135                 140

Val Arg Thr Ser Leu Leu Asp Ala Ser Asn Pro Glu Asn Tyr Trp Gln
145                 150                 155                 160

Ser Thr Lys Asn Leu Pro Leu Gln Asn Asn Ser Thr Thr Lys Asn Gly
                165                 170                 175

Lys Leu Arg Ile Glu Ala Pro Gly Phe Glu Phe Ser Ser Asn Ser Asn
            180                 185                 190

Asp Ser Ile Ser Thr Met Val Trp Ser Gly Ser Thr Asp His Tyr Glu
        195                 200                 205

Tyr Asn Leu Thr Val Lys Ala Thr Ser Arg Val Leu Leu Asn Ala Gly
    210                 215                 220

Thr Gly Tyr Phe Ser Trp Gly Asn Gly Asn Thr Ser Gln Trp Ser Leu
225                 230                 235                 240

Pro Ser Cys Gln Thr Ser Gly Met Leu Thr Leu Asp Asp Gln Ile Leu
                245                 250                 255

Gln Val Asp Glu Glu Arg Ser Met Thr Trp Tyr Asp Arg Gln Phe Gly
            260                 265                 270

Phe Gly Gly Val Asp Ser Glu Phe Thr Trp Phe Gly Val His Phe Pro
        275                 280                 285

Gly Ser Glu Val Lys Ala Ser Ile Trp Leu Ser Asp Asn Lys Asp Pro
    290                 295                 300

Ala Gln His Leu Arg Phe Ala Thr Val Arg Thr Asp Tyr Gly Leu Glu
305                 310                 315                 320

Ile Val Arg Phe Asn Val Thr Ile Glu Pro Glu Asn Ala Trp Thr Ser
                325                 330                 335

Pro Asn Ser Asn Tyr Thr Tyr Gln Thr Arg Trp Phe Leu Asp Phe Asp
            340                 345                 350

Asn Gly Asp Phe Leu Asp Ile Lys Ser Val Arg Asn Asp Gln Glu Ile
        355                 360                 365

Tyr Ala Lys Asn Ser Ile Phe Ser Ser Ser Pro Phe Thr Thr Val Lys
    370                 375                 380

Gly Arg Phe Phe Gly Gln Lys Thr Gly Phe Ala Leu Val Asp Ile Ser
385                 390                 395                 400

Pro Pro Thr Ser Leu Gln Gly Gly Asn Ala Val Phe
                405                 410

<210>  6
<211>  341
<212>  PRT
<213>  Streptosporangium roseum DSM 43021

<400>  6

Met Ala Phe Gly Thr Phe Ala Gly Ser Ala Ala Gln Ala Ala Ala Pro
1               5                   10                  15

Pro Ala Thr Pro Thr Ala Arg Ser Ala Thr Ser Gly Ile Pro Ala Leu
            20                  25                  30

Val Asp Pro Ala Ala Asp Leu Gly Arg His Ala Pro Gly Pro Lys Pro
        35                  40                  45

Thr Trp Phe Asp Ser Ile Tyr Phe Ile Ser Ser Val Lys Ala Asn Gly
    50                  55                  60

Gln Glu Phe Gly Leu Gln Val His Thr Arg Ile Leu Pro Ser Arg Ala
65                  70                  75                  80

Glu Ala Ala Tyr Arg Trp Thr Phe Ser Val Ile Asn Lys Thr Thr Gly
                85                  90                  95

Trp Tyr Lys Asp Tyr Asp Ala Met Val Lys Pro Glu Asp Tyr Arg Trp
            100                 105                 110

Ser Glu Gly Lys Leu Asp Ile Lys Ala Pro Gly Leu Ser Trp Thr Gly
        115                 120                 125

Asp Thr Val Arg Gln Ser Val Gln Val Glu Thr Pro Trp Gly Ser Leu
    130                 135                 140

Asp Val Gln Leu Ala Pro Thr Gly Pro Ala Met Asn Tyr Ala Ser Thr
145                 150                 155                 160

Gly Val Phe His Leu Ala Asp Gly Val Pro Asn Tyr Glu Phe Ala Leu
                165                 170                 175

Pro Glu Met Gln Thr Ser Gly Thr Leu Val Val Asp Gly Lys Lys His
            180                 185                 190

Lys Val Arg Gly Thr Ser Trp Leu Asp Arg Gln Trp Gly Glu Met Pro
        195                 200                 205

Thr Ser Leu Thr Arg Trp Thr Trp Met Asn Leu Ser Met Pro Asn Gly
    210                 215                 220

Asp Lys Val Ala Ile Trp Asp Thr Ile Gly Ala Glu Ser Lys Asp Ser
225                 230                 235                 240

Trp Ala Thr Val Leu His Pro Asp Gly Ser Tyr Asp Val Val Asp Val
                245                 250                 255

Glu Pro Leu Ala Gln Gly Ala Ser Arg Gln Trp Thr Ser Pro Ala Thr
            260                 265                 270

Gly Gln Arg Tyr Pro Thr Arg Trp Arg Ile Arg Ile Pro Ala Leu His
        275                 280                 285

Ser Glu Leu Asp Val Arg Val Thr Gly Asn Pro Asp Gln Glu Ile Gln
    290                 295                 300

Thr Pro Asp Gly Ser Gly Tyr Leu Glu Ala Thr Ala Ala Phe Thr Gly
305                 310                 315                 320

Lys Tyr Asn Gly Lys Lys Val Arg Gly Glu Asn Tyr Val Glu Met Thr
                325                 330                 335

Gly Glu Trp Thr Ser
            340

<210> 7
<211> 357
<212> PRT
<213> Burkholderia glumae (strain BGR1)

<400> 7

Met Phe Arg Phe Asn Leu His Asn Tyr Gly Arg Ala Ile Ala Val Ala
1               5                   10                  15

Val Leu Leu Ala Ser Ala Gly Pro Val Leu Ala Gln Thr Val Gly Thr
            20                  25                  30

Tyr Pro Pro Ile Glu Thr Ala Ile Ser Leu Pro Lys Asp Glu Thr Ser
        35                  40                  45

His Arg Gln Pro Val Glu Trp Trp Tyr Met Thr Gly His Leu Thr Gly
    50                  55                  60

Val Asp Pro Gln Gly Gly Gln Arg Gln Tyr Gly Phe Glu Leu Val Val
65                  70                  75                  80

Phe Gln Val Val Gly Val Ala Gly Leu Thr Pro Val Tyr Ser Ser His
                85                  90                  95

Phe Ala Ile Ser Asp Leu Asn Arg Gly Gln Phe Ser Phe Glu Glu Arg
            100                 105                 110

Thr Ala Pro Gly Gly Phe Ser Val Gln Pro Asp Arg Phe Asp Gln Asn
        115                 120                 125

Val Leu Gly Phe Gln Met Gly Gly Ser Ser Gly Gln Tyr Tyr Ala Lys
    130                 135                 140

Ala Ser Arg Ser Asp Leu Asp Tyr Ala Ile Asp Phe Lys Leu Thr Gln
145                 150                 155                 160

Lys Val Pro Val Thr Leu Asn Gly Ile Asn Gly Ile Glu Thr Met Gly
                165                 170                 175

Thr Phe Val Ser Pro Tyr Tyr Ser Tyr Thr Ala Leu Asp Thr Lys Gly
            180                 185                 190

Thr Leu Trp Asp His Gly Val Pro Val Gln Ile Thr Gly Gly Ala Thr
        195                 200                 205

Trp Tyr Asp His Glu Trp Ala Asn Ser Val Pro Gly Thr Asp Gly Gly
    210                 215                 220

Gly Trp Thr Trp Phe Gly Val Ser Met Ala Asp Asn Thr Gln Tyr Asn
225                 230                 235                 240

Leu Ser Phe Leu Gln Asp Ser Thr Gly Lys Val Val Lys Ala Ile Gly
                245                 250                 255

Val Lys Thr Ala Asn Gly Ser Tyr Ala Pro Ile Pro Glu Asp Gln Leu
            260                 265                 270

Ala Met Gln Glu Ile Gly Thr Trp Lys Ser Pro His Thr Gly Tyr Thr
        275                 280                 285

Tyr Ala Arg Ser Trp Arg Leu Thr Leu Pro Gly Gly Thr Ile Thr Val
    290                 295                 300

Val Pro Lys Ile Gln Asp Ser Glu Leu Ser Glu Thr Gly His His Tyr
305                 310                 315                 320

Tyr Tyr Glu Gly Pro Ala Ser Val Thr Gly Thr Ile Asn Gly Gln Pro
                325                 330                 335

Ala Thr Gly Asn Ala Tyr Ala Glu Val Asn Pro His Gly Val Glu Ser
            340                 345                 350

Gly Val Leu Leu Pro
        355

<210>  8
<211>  399
<212>  PRT
<213>  Candidatus Accumulibacter phosphatis clade IIA str.

<400>  8

Met Asp Arg Gly Arg Gly Ser Ser Asn Arg Val Arg Arg Ala Gly Ser
1               5                   10                  15

Gly Pro Ala Arg Pro Gly Ser Pro Arg Trp Phe Arg Arg Tyr Arg Trp
            20                  25                  30

Leu Ile Leu Ala Gly Trp Leu Leu Val Leu Leu Val Val Gly Trp Val
        35                  40                  45

Leu Phe Val Arg Ser Asp Lys Ala Ser Val Ala Pro Leu Pro Gly Gly
    50                  55                  60

Pro Ala Ala Glu Arg Ser Ala Pro Ser Ala Ala Arg Pro Asp Lys Met
65                  70                  75                  80

Pro Pro Val Lys Leu Pro Ala Asp Asp Ala Thr His Glu Asn Leu Thr
                85                  90                  95

Glu Trp Trp Tyr Tyr Ser Gly His Leu Gln Thr Glu Ser Gly Glu His
            100                 105                 110

Tyr Ser Phe His Leu Ala Ser Phe Met Arg Lys Gly Ser Phe Thr His
        115                 120                 125

Ala Ala Phe His Gly Ser Leu Leu Asp His Gln Asn Glu Lys Leu Tyr
    130                 135                 140

Thr Glu Gln Ala Arg Thr Ala Gly Asn Pro Ser Asp Gly Arg Arg Asp
145                 150                 155                 160

Gly Phe Asp Phe Ser Phe Gly Asp Trp Arg Leu Gln Gly Val Gly Ala
                165                 170                 175

Gln His Lys Ala Arg Met Ala Gly Lys Asp Phe Ser Leu Asp Leu Gln
            180                 185                 190

Met Ser Asp Asn Arg Pro Pro Val Leu His Gln Ala Pro Gly Thr Pro
        195                 200                 205

Val Ala Gly Leu Leu Asp Leu Gly Ala Ser Gly Thr Ser Tyr Tyr Thr
    210                 215                 220

Ser Arg Pro Arg Met Ser Ala Gln Gly Thr Leu Thr Ile Ala Gly Thr
225                 230                 235                 240

Ala Lys Ala Val Arg Gly Glu Val Trp Phe Asp His Gln Trp Gly Asp
                245                 250                 255

Phe Glu Ala Ala Glu Leu Arg Trp Asn Trp Phe Ala Leu Gln Leu Thr
            260                 265                 270

Asp Gly Ala Glu Leu Met Ile Phe Glu Ile Phe Glu Arg Arg Ser Gly
        275                 280                 285

Ala Ser Val Leu Arg Met Gly Thr Tyr Val Lys Asp Gly Ala Val Ser
    290                 295                 300

Ala Leu Gly Ala Ala Asp Phe Glu Ala Thr Ser Arg Gly Thr Trp Lys
305                 310                 315                 320

Ser Pro Ser Ser Gly Val Val Tyr Pro Ile Leu Trp Thr Ile Ser Val
                325                 330                 335

Pro Ser Arg Gly Leu Lys Leu Lys Val Asp Pro Val Ile Arg Trp Ser
            340                 345                 350

Gln Phe Asp Ala Arg Thr Thr Thr Leu Asn Val Tyr Trp Glu Gly Ala
        355                 360                 365

Val Lys Leu Ser Gly Ser His Thr Gly Val Gly Phe Met Glu Leu Asn
    370                 375                 380

Gly Tyr Thr Met Pro Glu Glu Gln Lys Ala Asn Asn Arg Lys Pro
385                 390                 395

<210> 9

<211>  363
<212>  PRT
<213>  Neosartorya fischeri (strain ATCC 1020 / DSM 3700 / FGSC A1164 / NRRL
181)

<400>  9

Met Arg Ser Leu Leu Val Ala Leu Val Thr Phe Leu Ser Ala Val Gly
1               5                   10                  15

Gly Leu Glu Val Pro Ser Phe Ala Pro Pro Lys Phe Pro Ala Ser Thr
            20                  25                  30

Ile Asp Asn Asp Gly Thr His Ala Gly Arg Tyr Asp Val Arg Lys Thr
        35                  40                  45

Met Val Trp Leu Asp Glu Glu Asn Pro Asp Asn Leu Asn Ser Tyr Phe
    50                  55                  60

Phe Leu Phe Trp Leu Thr Thr Val Asp Gly Arg Lys Tyr His Val Thr
65                  70                  75                  80

Leu Ala Pro Asn Phe Phe Arg Gly Ser Thr Thr Gly Ile Phe Arg Val
                85                  90                  95

Glu Asp Leu Gln Asp Leu Ser Ser Thr Gly Ala Thr Val Phe Ala Pro
            100                 105                 110

Gly Thr Gly Ser Thr Glu Phe Leu Asp Phe Arg Ser Glu Ile Gln Asn
        115                 120                 125

Leu Thr Ser Pro Arg Gly Ser Asp Asn Tyr Thr Asp Leu Thr Ile Ser
    130                 135                 140

Thr Asn Tyr Ala Gly Val Pro Leu Asn Val His Ile Thr Pro Thr Gly
145                 150                 155                 160

Lys Asn Leu Tyr Val Gly Gly Gly Gly Gly Ile Thr Leu Ser Ser Asp
                165                 170                 175

Gly Asn Asp Phe Arg Arg Val Ile Pro Gly Tyr Ser Trp Tyr Trp Gly
            180                 185                 190

Asn Pro Thr Leu Arg Leu Asn Gly Thr Leu Thr Val Asn Gly Glu Glu
        195                 200                 205

Ile Ala Ile Asp Cys Asp Gln Ser Arg Gly Tyr Phe Glu Arg Gln Trp
    210                 215                 220

Gly Glu Phe Gly Ile Ser Gly Gly His Tyr Gly Tyr Trp Leu Tyr Leu
225                 230                 235                 240

Ser Asn Gly Leu Met Ile His Gly Trp Val Val Gly Pro Thr Ile Glu
                245                 250                 255

Gln Pro Phe Gly Val Pro Ala Trp Ala Thr Val Trp His Pro Asn Gly
            260                 265                 270

Val His Glu Val Val Glu Val Gly Lys Asn Thr Thr Ala Phe Asp Val
        275                 280                 285

Trp Lys Ser Glu Tyr Ser Gly Leu Asp Tyr Phe Lys Gln Val Lys Leu
    290                 295                 300

Asp Ile Pro Thr Arg Gln Ala Gly Phe Asn Ile His Gln Leu Ile Arg
305                 310                 315                 320

Glu Ser Glu Leu Arg Pro Leu Ser Gly Thr Asp Gly Tyr Asn Ile Ser
                325                 330                 335

Glu Ala Tyr Ala Gln Gly Glu Gly Val Trp Glu Gly Glu His Val Thr
            340                 345                 350

Phe Phe Gly His Leu Glu Gln Leu Ser Tyr Trp
        355                 360

<210>  10
<211>  315
<212>  PRT
<213>  Penicillium chrysogenum (strain ATCC 28089 / DSM 1075 / Wisconsin 54-
1255)

<400>  10

Met Val Trp Leu Asp Glu Glu Asn Pro Asn Asn Leu Asn Ser Tyr Phe
1               5                   10                  15

Phe Leu Phe Trp Phe Thr Thr Met Asp Gly Arg Lys Tyr His Val Thr
            20                  25                  30

Leu Ala Pro Asn Phe Phe Arg Gly Arg Thr Thr Gly Ile Phe Arg Val
        35                  40                  45

Glu Asp Leu Gln Asp Leu Ser Ser Thr Gly Ala Thr Val Phe Ala Pro
    50                  55                  60

Gly Thr Gly Ser Asn Glu Phe Leu Asp Phe Arg Ser Glu Ile Gln Asn
65                  70                  75                  80

Leu Thr Ser Pro Arg Val Ser Asp Asn Tyr Thr Asp Leu Asn Val Ser
                85                  90                  95

Thr Asn Tyr Ala Gly Val Gln Leu Asn Val His Ile Thr Pro Thr Gly
            100                 105                 110

Lys Asn Leu Tyr Ile Gly Gly Gly Gly Gly Ile Thr Leu Ser Ser Asp
        115                 120                 125

Asp Asn Asp Phe Arg Arg Ile Ile Pro Gly Tyr Ser Trp Tyr Trp Gly
    130                 135                 140

Asn Pro Thr Leu Arg Leu Asn Gly Thr Ile Thr Ile Asp Gly Glu Glu
145                 150                 155                 160

Ile Ala Val Asp His Asp Gln Ser Arg Gly Tyr Phe Glu Arg Gln Trp
                165                 170                 175

Gly Glu Phe Gly Ile Ser Gly Gly His Tyr Gly Tyr Trp Leu Tyr Leu
            180                 185                 190

Ser Asn Gly Leu Met Ile His Gly Trp Val Val Ser Pro Thr Thr Glu
        195                 200                 205

Gln Pro Phe Gly Val Pro Ala Trp Ala Thr Val Trp His Pro Asn Gly
    210                 215                 220

Val His Glu Val Val Glu Val Gly Lys Asn Thr Thr Ala Phe Asp Val
225                 230                 235                 240

Trp Lys Ser Glu Tyr Ser Gly Arg Asn Tyr Phe Asn Gln Val Glu Leu
                245                 250                 255

Asp Ile Pro Ser Arg Gln Ala Arg Phe Asp Ile His Gln Leu Ile Arg
            260                 265                 270

Glu Ser Glu Val Arg Pro Leu Ser Gly Thr Asp Gly Tyr Asn Ile Ser
        275                 280                 285

Glu Ala Tyr Ala Gln Gly Gln Gly Val Trp Glu Gly Glu Pro Val Thr
    290                 295                 300

Phe Phe Gly His Leu Glu Gln Leu Ser Phe Trp
305                 310                 315

<210>  11
<211>  747

<212>  PRT
<213>  Trypanosoma cruzi

<400>  11

Met Arg Asn Phe Ser Gly Phe Gly Lys Lys Gly Glu Asn Gly Gly Asn
1               5                   10                  15

Ser Ile Glu Leu Leu Gly Glu Pro Leu Leu Lys Pro Ser Met Met Asp
            20                  25                  30

Ile Ile Ser His Glu Ala Phe Leu Gln Trp Trp Tyr Phe Asn Ala His
        35                  40                  45

Leu Arg Asp Val Asp Ser Glu Lys Glu Phe Ser Phe Phe Thr Ser Phe
    50                  55                  60

Phe Ala Phe Lys Asp Pro Lys Lys Glu Leu Lys Gly Asp Phe Tyr Asp
65                  70                  75                  80

Ala Cys Thr Trp Ala Leu Ile Asp Val Thr Glu Lys Lys Tyr Tyr Pro
                85                  90                  95

Asp Ser Leu Leu Asp Arg Arg Ala Ala Glu Ile Ile Ile Gly Lys Ile
            100                 105                 110

Glu Ala Asn Glu Ala Lys Tyr Asn Ile Asn Glu Tyr Pro Val Glu Ile
        115                 120                 125

Ile Lys Glu Leu Leu Lys Gln Gly Lys Val Pro Leu Pro Asp Arg Leu
    130                 135                 140

Met Glu Lys Thr Ala Val Tyr Asp Asn Asp Thr Leu Lys Ile Asn Phe
145                 150                 155                 160

Asn Asp Glu Phe Phe Leu Glu Gly Glu Gly Asp Asp Ala Glu Arg Arg
                165                 170                 175

Tyr Lys Leu His His His Asn Pro Leu Arg Asn Ile Thr Val Glu Leu
            180                 185                 190

Glu Phe Thr Ala Arg Thr Ile Pro Ser Phe His Gly Asp Asn Gly Phe
        195                 200                 205

Ile Lys Arg Met Phe Tyr Tyr Tyr Tyr Pro Ser Met Ser Val Glu Gly
    210                 215                 220

Lys Leu Thr Ile Gly Asp Ser Thr Ile Asn Val Lys Gly Asp Gly Trp
225                 230                 235                 240

Tyr Asp Arg Glu Tyr Gly Ser Pro Ala Asp Asp Ser Arg Glu Asn His
                245                 250                 255

Gly Ile Trp Asn Trp Phe Gly Leu Gln Leu Ser Asn Gly Tyr Gln Leu
            260                 265                 270

Asn Leu Phe Glu Leu Phe Asp Gly Ile Thr Gly Lys Arg Glu Glu Gln
        275                 280                 285

Leu Ala Val Leu Thr Ser Gly Glu Lys Arg Tyr Thr Cys Arg Asp Phe
    290                 295                 300

Ile Ile Glu Pro Val Glu Cys Trp Thr Ser Leu Val Thr Phe Gln Asp
305                 310                 315                 320

Tyr Asn Val Ala Phe His Ile Glu Leu Pro Ser Phe Gln Leu Ser Phe
                325                 330                 335

Asp Leu Arg Ala Ser Phe Asn His Gln Glu Phe Gln Thr Val Leu Thr
            340                 345                 350

Phe Phe Gly Phe Tyr Glu Gly Arg Val Thr Gly Glu Gly Ser His Glu
        355                 360                 365

Gly Thr Pro Val Ser Ile Ile Gly Phe Tyr Glu Gln Lys Asn Ala Ser
    370                 375                 380

Ser Pro Ala Ser Thr Phe Ser Ile Ile Asn Lys Leu Gly Glu Lys Thr
385                 390                 395                 400

Arg Gly Ile Leu Ser Glu Val Tyr Pro Leu His Ala Ser Val Glu Trp
                405                 410                 415

Ile Glu Lys Asn Val Leu Gly Arg Phe Cys Thr Cys Lys Gly Val Leu
            420                 425                 430

Pro Gln His Ile Cys Asp Thr Leu Phe Leu Pro Val Arg Phe Leu Ile
        435                 440                 445

Asp Arg Gly Gly Lys Cys Trp Arg Thr Phe Phe Leu Phe Ala Cys Ser
    450                 455                 460

Asn Ala Leu Ser Arg Asp Tyr Ala Asp Thr Ser Arg Cys Val Ile Ile
465                 470                 475                 480

Thr Glu Leu Gly His Val Gly Ser Leu Ile Ile Asp Asp Ile Glu Asp

                485                 490                 495

Gly Ser Ser Val Arg Arg Gly Gly Lys Ala Ala His Val Glu Phe Gly
            500                 505                 510

Thr Pro Thr Ala Ile Asn Ala Gly Thr Ala Cys Phe Phe Met Ser Val
        515                 520                 525

Pro Phe Ala Asp Val Ser Ser Leu Pro Ala Glu Lys Ala Lys Arg Phe
    530                 535                 540

Tyr Glu Leu Tyr Phe Glu Met Met Lys Ala Ala His Ala Gly Gln Gly
545                 550                 555                 560

Leu Asp Ile Arg Gly Leu Gly Tyr Leu Met Pro Asp Val Val Arg Thr
                565                 570                 575

Gly Glu Thr Lys Ala Leu Glu Asp Ala Leu Lys Ala Ile His Thr Cys
            580                 585                 590

Lys Thr Gly Ala Ile Cys Gly Ala Ile Cys Ser Phe Ala Cys Val Leu
        595                 600                 605

Thr Asp Thr Ser Glu Glu Val Thr Leu Ala Met Glu Glu Phe Gly Lys
    610                 615                 620

Ala Ile Gly Leu Ala Tyr Gln Ile Ile Asp Asp Val Leu Gly Val Lys
625                 630                 635                 640

Gly Phe Glu Gly Asn Leu Lys Glu Ala Gly Glu Asp Ile Arg Asp Gly
                645                 650                 655

Lys Ile Thr Tyr Pro Val Ala Lys Ala Met Gly Lys Leu Glu Leu Pro
            660                 665                 670

Gln Arg Glu Arg Ile Trp Ser Ile Leu Asn Glu Arg Thr Ser Asp Arg
        675                 680                 685

Glu Lys Ile Glu Glu Ala Leu Ser Leu Ile Asn Ser Ala Asn Val Val
    690                 695                 700

Asp Glu Cys Val Ala Asp Ala Arg Gln Leu Val Glu Glu Glu Trp Lys
705                 710                 715                 720

Val Val Asp Pro Leu Ile Pro Asp Thr Val Ser Lys Val Met Leu Arg
                725                 730                 735

Ala Leu Cys Phe Tyr Ile Ile Arg Arg Asn Cys
            740                 745